**Europäisches Patentamt**

**European Patent Office**

**Office européen des brevets**

(11) Publication number : **0 542 679 A1**

## (12) EUROPEAN PATENT APPLICATION

(21) Application number : **92810845.5**

(22) Date of filing : **03.11.92**

(51) Int. Cl.⁵ : **C12N 15/12, C12N 15/62, A61K 37/02, C12N 1/21, // (C12N1/21, C12R1:865)**

Declaration under Rule 28(4) EPC (expert solution)

(30) Priority : **11.11.91 EP 91810870**

(43) Date of publication of application : **19.05.93 Bulletin 93/20**

(84) Designated Contracting States :
**AT BE CH DE DK ES FR GB GR IE IT LI LU NL PT SE**

(71) Applicant : **CIBA-GEIGY AG**
**Klybeckstrasse 141**
**CH-4002 Basel (CH)**

(72) Inventor : **McMaster, Gary Kent, Dr.**
**Violaweg 70**
**CH-4303 Kaiseraugst (CH)**
Inventor : **Cox, David, Dr.**
**Hauptstrasse 245**
**CH-4204 Himmelried (CH)**
Inventor : **Cerletti, Nico, Dr.**
**Rütistrasse 11**
**W-4103 Bottmingen (CH)**
Inventor : **Kuhla, Jochen, Dr.**
**Johann-Schillstrasse 3**
**W-7806 Buchheim (DE)**

(54) **Novel hybrid transforming growth factors.**

(57)    The invention relates to novel hybrid TGF-βs, a process for the production of biologically active, dimeric hybrid proteins and pharmaceutical compositions comprising them. Hybrid TGF-βs produced by this process can be used in various therapeutic modalities such as for the promotion and acceleration of wound healing and bone and tissue repair, the treatment of cancer, as a bone marrow protective agent, mediator of cardioprotection, anti inflammatory or immunosuppressive agent or as a growth regulator in mammalian cell cultures.

EP 0 542 679 A1

## Novel hybrid Transforming Growth Factors

The invention relates to novel recombinant hybrid TGF-βs, a process for the production of the recombinant proteins and a process for the preparation of biologically active, dimeric hybrid proteins and pharmaceutical compositions comprising them.

## Background of the invention

TGF-β was originally purified to homogeneity from human platelets, human placenta and bovine kidney and identified as a homodimeric protein with a molecular mass of 25.000 D. First characterized by its ability to act synergistically with EGF or TGF-α to induce anchorage-independent growth of untransformed NRK cells, recently, TGF-β has been shown to exhibit numerous regulatory effects on a wide variety of both normal and neoplastic cells indicating the importance of this protein as a multifunctional regulator of cellular activity. TGF-β may either stimulate mitogenesis, cell proliferation and growth, or may effectively inhibit said processes, or may exhibit other actions like e.g. control of adipogenesis, myogenesis, chondrogenesis, osteogenesis und immune cell function, stimulation of chemotaxis, or induction or inhibition of differentiation depending upon the cell or tissue type, and the presence or absence of other growth factors. Many of the actions of TGF-β are related to the response of cells or tissues to stress or injury, and to the repair of resultant damage. After inflammation, TGF-β plays the major role in the formation of granulation tissue, increases the expression of genes associated with extracellular matrix formation such as fibronectin, collagen and several protease inhibitors and stimulates collagen-matrix contraction by fibroblasts, suggesting its possible role in connective tissue contraction.

There are five known distinct isoforms of TGF-β with 64-82% identity. Until now, three distinct types of TGF-βs designated as TGF-β1, TGF-β2 and TGF-β3 thus far have been demonstrated to be expressed in mammalian tissues. TGF-β4 has been described only in chicken and TGF-β5 only in frog. TGF-β1, TGF-β2 and TGF-β3 have been cloned and characterized by sequence analysis. They are synthesized as 390 to 412 amino acids long inactive precursors consisting of a signal sequence, of the Latency Associated Protein LAP and the 112 amino acids long TGF-β sequence. In their mature, biologically active forms, TGF-βs are acid- and heat-stable disulfide-linked homodimers of two polypeptide chains of 112 amino acids each.

The complete amino acid sequences of human, murine and simian TGF-β1 show remarkable sequence conservation, differing only in a single amino acid residue. Comparison of the amino acid sequence of human TGF-β1, human TGF-β2 and human TGF-β3 has demonstrated that the three proteins exhibit in their mature forms about 70-80 % sequence identity. A heterodimeric TGF-β1.2 has been isolated from porcine platelets and consists of one subunit of TGF-β1 disulfide-linked to one subunit of TGF-β2.

Natural sources (e.g. platelets) are inadequate to supply the amounts required for clinical studies and expected therapeutic usage. Therefore, attempts have recently been undertaken aiming to produce TGF-βs by means of recombinant techniques. However, it has proven to be extremely difficult to synthesize recombinant TGF-β while retaining its biological activity. TGF-β1, TGF-β2 and TGF-β3 contain 9 cysteine residues each, at least some of which are involved in intrachain and interchain disulfide bond formation which results in the complex tertiary structure of the biologically active, dimeric molecules. Heterologous expression of TGF-β may lead to a product which, although having the correct primary structure, fails to fold properly to produce the correct secondary or tertiary structures and which, therefore, lacks the biological activity. To date, the secondary and tertiary structures of TGF-βs are unknown.

Taking the complexity of the native TGF-β molecules into account, it has generally been considered expedient to express the respective TGF-β genes in cells derived from higher organisms.

However, following expression of full-length DNAs encoding the precursor forms of TGF-βs (390-414aa) in eukaryotic systems, yields of biologically active, correctly folded material remain far from satisfactory. Only low levels of expression have described for TGF-β1, TGF-β2 and TGF-β3 in CHO cells amplified with Methotrexate (MTX), e.g. expression of 6 μg/ml TGF-β1 at 20μM MTX concentration (Gentry, L. E. et al., (1987) Molec.Cell.Biol. 7:3418-3427), expression of 5 μg/ml TGF-β2 at 10μM and 50μM MTX concentration (Madisen, L. et al.,(1990) Growth Factors 3:129-138), expression of 2 μg/ml TGF-β3 at 1.6mM MTX concentration (Graycar, J.L. et al., (1989) Mol. Endocrinol. 3:1977-1986) and 30 ng/ml at 20mM MTX concentration (Ten Dijke, P. et al., (1990) Annals of the New York Academy of Sciences 593:26-42). In all 3 cases the TGF-β is secreted in a latent form since acidification is necessary for detection of biological activity.

EP-A-0 433 225 describes a microbial-based process for the production of biologically active, dimeric TGF-β-like protein from its denatured or otherwise non-native form. Considerable amounts of TGF-β can be obtained when the monomeric form is subjected to refolding.

Different TGF-β isoforms have different potencies and/or activities in various systems. For example,

- TGF-β3 is 10-fold more active than TGF-β2 in inducing mesoderm formation in Xenopus laevis. TGF-β1 is inactive in this assay.
- TGF-β1 is 10 to 100-fold more potent than TGF-β2 in inhibiting DNA synthesis in bovine aortic endothelial cells.
- TGF-β2 and TGF-β3 are markedly more potent than TGF-β1 in stimulating proliferation of AKR-2B fibroblasts and in inhibiting DNA synthesis in CCL64 Mink Lung Epithelial cells. TGF-β1 is markedly more potent than TGF-β2 or -3 in inhibiting the proliferation of the mammary carcinoma cell line MCF-7. TGF-β3 is markedly more potent than TGF-β1 or -2 in inhibiting DNA synthesis in mouse and human keratinocytes.
- TGF-β2 is markedly more active in vivo, in stimulating bone formation than TGF-β1.

A need exits for hybrid TGF-β molecules with altered or mixed activities as compared with the parent molecules which hybrid TGF-βs exhibit improved activities and/or novel or advantageous properties in certain biological systems.

## Object of the invention

It is an object of the present invention to provide novel hybrid TGF-β molecules, recombinant DNA molecules encoding same, hybrid vectors comprising such recombinant DNA molecules, transformed hosts suitable for the multiplication and/or expression of the recombinant DNA molecules, and processes for the preparation of the hosts, DNA molecules and hybrid TGF-β molecules.

## Detailed description of the invention

The invention concerns novel hybrid TGF-β molecules, preferentially such having biological TGF-β activity. "Biological activity" for the purpose herein is defined as either

(a) the cell migration promoting activity on normal Balb/c 3T3 fibroblasts, which can be measured by counting the number of cells that migrate into a "wounded" mono-layer culture of said cells, in the presence of a serum-free medium containing the TGF-β-like protein, as compared to the number of cells that migrate in the absence of the TGF-β-like protein, or

(b) the growth promoting activity on normal Balb/c 3T3 fibroblasts determined by the stimulatory effect of the TGF-β-like protein on cellular DNA synthesis and cell division, or

(c) the growth inhibition of A375 melanoma cells determined by a colorimetrical assay which reflects the number of cells treated with the TGF-β-like protein for a given culture period as compared to the number of non-treated cells, or

(d) the accelerated healing of partial-thickness burn wounds, by a process of re-epithelialization, in old mice following multiple topical applications of the TGF-β-like protein as compared to untreated control wounds, or

(e) the accelerated healing of full-thickness incisional wounds, as determined by tensile strength measurements and the histological analyses of biopsies, in adult rats following single topical applications of the TGF-β-like protein as compared to untreated control wounds, or

(f) the increase in formation of fibrous granulation tissue, together with a marked increase in vascularity of the said tissue, both in and around porous wound-chamber implants in adult rats following multiple local injections of the TGF-β-like protein into the chamber as compared to untreated control chambers.

A substantial feature of the said novel hybrid TGF-β molecules is that they consist of two or more, preferentially of 2, 3, 4, 5 or 6, more preferably of two parts, the said parts consisting of contiguous stretches of 6 or more amino acids of a sequence which occurs in the mature part in two or more, preferentially in two or three, more preferably in two, of the TGF-β isoforms defined hereinafter. In all cases the stretches of amino acid residues in the hybrid molecules correspond to contiguous stretches of an equal length which occur at the corresponding locations in the primary amino acid sequence of a different TGF-β isoform.

The term "TGF-β-isoform" is intended to embrace TGF-β1, TGF-β2 and TGF-β3 of mammalian such as human or animal origin, e.g. simian, murine, porcine, equine or bovine, and TGF-β4 of chicken and TGF-β5 of frog. It preferentially includes TGF-β1, TGF-β2, and TGF-β3, most preferentially human TGF-β1, human TGF-β2 and human TGF-β3 with the amino acid sequences depicted the Sequence Listing under SEQ ID NO. 1 to 3.

The preferred type of hybrid proteins of the invention comprises two parts, each consisting of contiguous amino acid stretches from different TGF-β isoforms. More preferred hybrid TGF-β molecules are those consisting of a N-terminal part of TGF-β1 and a C-terminal part of TGF-β2, a N-terminal part of TGF-β2 and a C-terminal part of TGF-β1, a N-terminal part of TGF-β1 and a C-terminal part of TGF-β3, a N-terminal part of TGF-β3 and a C-terminal part of TGF-β1, a N-terminal part of TGF-β2 and a C-terminal part of TGF-β3, or a N-terminal part of TGF-β3 and a C-terminal part of TGF-β2. Most preferably, these hybrid TGF-β molecules are composed of parts of TGF-β1, TGF-β2 and/or TGF-β3 sequences shown in the Sequence listing under SEQ ID NO. 1 to 3.

Preferred hinge point between the amino acid stretches derived from the parent TGF-β isoforms are between amino acids 56 and 57, 79 and 80, 90 and 91, 22 and 23, or between 44 and 45 (numbering relates to the amino acids numbers of human TGF-β, starting at the N-terminus). Thus, preferred hybrids of the present invention are composed of the 56, 79, 90, 22 or 44 N-terminal amino acids of the TGF-β isoform providing the N-terminal part of the hybrid and of the 56, 33, 22, 90, or 68, respectively, C-terminal amino acids of the TGF-β isoform providing the C-terminal part of the hybrid molecule.

Preferred forms of the hybrid TGF-β molecules composed of parts of TGF-β1, TGF-β2 and/or TGF-β3 sequences shown in the Sequence listing under SEQ ID NO. 1 to 3 are hybrid proteins having the hinge points between amino acids 44 and 45 and consisting of the 44 N-terminal amino acids of TGF-β1 and of the 68 C-terminal amino acids of TGF-β2, of the 44 N-terminal amino acids of TGF-β2 and of the 68 C-terminal amino acids of TGF-β1, of the 44 N-terminal amino acids of TGF-β1 and of the 68 C-terminal amino acids of TGF-β3, of the 44 N-terminal amino acids of TGF-β3 and of the 68 C-terminal amino acids of TGF-β1, of the 44 N-terminal amino acids of TGF-β2 and of the 68 C-terminal amino acids of TGF-β3, or even more preferably of the 44 N-terminal amino acids of TGF-β3 and of the 68 C-terminal amino acids of TGF-β2. These hybrids are named TGF-β1(44/45)β2, TGF-β2(44/45)β1, TGF-β1(44/45)β3, TGF-β3(44/45)β1, and TGF-β2(44/45)β3, respectively. The even more preferred form is accordingly named TGF-β3(44/45)β2. The amino acid sequences of these hybrids as well as DNA sequences encoding them are depicted in the sequence listing (SEQ ID NOs. 4 to 9).

Preferred forms of the hybrid TGF-β molecules composed of parts of TGF-β1, TGF-β2 and/or TGF-β3 sequences shown in the Sequence listing under SEQ ID NO. 1 to 3 are also hybrids consisting of the TGF-β1 N-terminal and TGF-β2 C-terminal halves, i.e. the 56 N-terminal amino acids of TGF-β1 and the 56 C-terminal amino acids of TGF-β2, of the TGF-β2 N-terminal and TGF-β1 C-terminal halves, of the TGF-β1 N-terminal and TGF-β3 C-terminal halves, of the TGF-β3 N-terminal and TGF-β1 C-terminal halves, of the TGF-β2 N-terminal and TGF-β3 C-terminal halves, and of the TGF-β3 N-terminal and TGF-β2 C-terminal halves. These hybrids are named TGF-β1(56/57)β2, TGF-β2(56/57)β1, TGF-β1(56/57)β3, TGF-β3(56/57)β1, TGF-β2(56/57)β3, and TGF-β3(56/57)β2, respectively.

Likewise, preferred forms of the hybrid TGF-β molecules composed of parts of TGF-β1, TGF-β2 and/or TGF-β3 sequences shown in the Sequence listing under SEQ ID NO. 1 to 3 are also hybrids having the hinge point between amino acid 79 and 80. In accordance with the nomenclature explained hereinbefore they are named TGF-β1(79/80)β2, TGF-β2(79/80)β1, TGF-β1(79/80)β3, TGF-β3(79/80)β1, TGF-β3(79/80)β2, and TGF-β2(79/80)β3.

Likewise, preferred forms of the hybrid TGF-β molecules composed of parts of TGF-β1, TGF-β2 and/or TGF-β3 sequences shown in the Sequence listing under SEQ ID NO. 1 to 3 are also the hybrids TGF-β1(90/91)β2, TGF-β2(90/91)β1, TGF-β1(90/91)β3, TGF-β3(90/91)β1, TGF-β3(90/91)β2, and TGF-β2(90/91)β3, all having the hinge point between amino acid 90 and 91.

Likewise, preferred forms of the hybrid TGF-β molecules composed of parts of TGF-β1, TGF-β2 and/or TGF-β3 sequences shown in the Sequence listing under SEQ ID NO. 1 to 3 are also the hybrids TGF-β1(22/23)β2, TGF-β2(22/23)β1, TGF-β1(22/23)β3, TGF-β3(22/23)β1, TGF-β3(22/23)β2, and TGF-β2(22/23)β3, all having the hinge point between amino acid 22 and 23.

The amino acid sequences of the preferred hybrids and the nucleotide sequences encoding them can be easily deduced from the amino acid sequences of the parent TGF-β1, -β2, and -β3 molecules and the corresponding DNA sequences given in the sequence listing under SEQ ID NO. 1 to 3.

The invention also concerns recombinant DNA molecules encoding hybrid TGF-β molecules of the invention. Preferred DNA molecules are those which are coding for the preferred hybrid proteins.

The nucleotide sequences encoding the TGF-β isoforms specified above are known from literature or can be deduced from the amino acid sequences of the proteins according to conventional rules. Starting from the nucleotide sequences encoding the parent TGF-β isoform molecules, a DNA molecule encoding any desired hybrid molecule can be deduced and constructed according to conventional methods known in the art including, but not limited to, the use of polymerase chain reaction (PCR) technology, DNA restriction enzymes, synthetic oligonucleotides, DNA ligases and DNA amplification techniques. Alternatively, the coding sequences may be synthesized in whole or in part using chemical methods known in the art.

The DNA coding for the TGF-β isoforms, e.g. for human TGF-β1, -2 and -3, i.e. the parent molecules of preferred hybrids of the invention, may be obtained from human cell sources by conventional methods, e.g. by applying cDNA technology, from vectors available in the art or by chemical synthesis of the DNA.

4

A recombinant DNA molecule encoding hybrid TGF-β molecules of the invention also comprises DNA sequences which are degenerate within the meaning of the genetic code in that an unlimited number of nucleotides are replaced by other nucleotides without changing the amino acid sequence for which they code. Such degenerate DNA sequences may be useful because of the occurence of different restriction patterns or because of preferred codon usage in a particular host.

The invention also concerns hybrid vectors comprising a DNA sequence encoding a hybrid TGF-β molecule of the invention. The hybrid vectors of the invention provide for replication and optionally expression of the DNA encoding a hybrid of the invention, either as an extrachromosomal element or by integration into the host genome. Several possible vector systems are available, and the vector selected is depending on the host cells envisaged for transformation.

A hybrid vector of the invention comprises a DNA sequence encoding a hybrid TGF-β molecule of the invention linked with an origin of replication allowing the replication of the vector in the host cell, or a functionally equivalent sequence, e.g. an autonomously replicating sequence ARS from yeast. Another type of vector, i.e. an expression vector, comprises a DNA sequence encoding a hybrid TGF-β molecule of the invention operably linked with expression control sequences, e.g. promoters, which ensure the effective expression of the hybrid TGF-β proteins in a transformed host, and an origin of replication allowing the replication of the vector in the host cell, or a functionally equivalent sequence. However, in case that the vector of the invention is a so-called integrative vector, i.e. it is integrated into a host chromosome after transformation and is replicated as part of the chromosome, it does not necessarily comprise an origin of replication or functionally equivalent sequence.

Suitable hybrid vectors may be derived from any vector useful in the art of genetic engineering, such as from viruses, phages, cosmids, plasmids or chromosomal DNA. Examples of vectors that are suitable for the expression of the hybrid TGF-β protein in an E. coli strain are bacteriophages, for example derivatives of the bacteriophage λ or M13 or plasmids, such as the plasmid pBR322 and its derivative pPLMu. Examples of vectors that are suitable for the expression of the hybrid TGF-β protein in a yeast strain are 2μ based plasmids. Vectors suitable for the expression in higher eukaryotic cells are vectors based on the insect Baculovirus or on other viruses, such as SV40, Herpes viruses, Papillomaviruses, Retroviruses and the like. Suitable vectors may contain a marker gene, which renders possible the selection and identification of the microorganisms transformed by the expression plasmids by means of a phenotype feature. Suitable marker genes impart to the microorganism, for example, resistance to heavy metals, antibiotics such as ampicillin or tetracyclin, and the like.

Several promoters can be used for regulating the expression of hybrid TGF-β proteins in E. coli. Especially promoters of strongly expressed genes are used. Suitable promoters are the E. coli lac, tac, trp and lpp promoters, furthermore the phage λN or the phage λpL promoter, and others.

Vectors suitable for replication and expression in S. cerevisiae contain a yeast-replication origin and a selective genetic marker for yeast. Hybrid vectors that contain a yeast replication origin, for example the chromosomal autonomously replicating segment (ars), are retained extrachromosomally within the yeast cell after transformation and are replicated autonomously during mitosis. Also, hybrid vectors that contain sequences homologous to the yeast 2μ plasmid DNA can be used. Such hybrid vectors are integrated by recombination in 2μ plasmids already present within the cell, or replicate autonomously. Suitable marker genes for yeast are especially those that impart antibiotic resistance to the host or, in the case of auxotrophic yeast mutants, genes that complement the host lesions. Corresponding genes impart, for example, resistance to the antibiotic cycloheximide or provide for prototrophy in an auxotrophic yeast mutant, for example the URA3, LEU2, HIS3 or the TRPI gene.

Promoters suitable for expression in yeast are, for example, those of the ADHI, ADHII, or PH05 gene, and also promoters involved in glycolysis, for example the PGK or the GAP promoter.

Optionally, signal sequences which allow the secretion of the hybrid TGF-β protein can be included in the expression vector. Suitable signal sequences are e.g. derived from the yeast acid phosphatase (PHO5) or the yeast invertase gene.

The present invention also concerns a method for the preparation of the DNA molecules of the invention defined hereinbefore or in the examples. They are prepared according to conventional methods, e.g. by means of restriction enzymes, ligases, phosphatases or polymerases, or by applying polymerase chain reaction (PCR) techniques, or by conventional chemical synthesis, or by isolating the desired DNA molecules from natural sources or transformed hosts, e.g. by a method comprising culturing a host transformed with a vector providing for the replication of the desired DNA molecule and isolating the DNA molecule from the host by conventional methods, e.g. by extraction with phenol and/or chloroform.

Microbial hosts comprising a nucleotide sequence encoding the hybrid TGF-β protein linked in the proper reading frame to an expression control sequence can be prepared by recombinant DNA techniques which are well known in the art and which comprise the steps of

- preparing a hybrid vector comprising a DNA sequence encoding the hybrid TGF-β protein under the expression control of a suitable expression control sequence,
- transforming said microbial host with said hybrid vector, and
- selecting transformed microbial host cells from untransformed host cells.

The selection of a suitable vector is determined by the microbial host cell provided for the transformation.

Suitable microbial hosts for performing the present invention are yeast strains as Saccharomyces cerevisiae or bacteria such as Bacillus subtilis or preferentially Escherichia coli.

The transformed microbial hosts are cultured in a liquid medium containing assimilatable sources of carbon, nitrogen and inorganic salts, applying methods known in the art.

Various carbon sources are usable. Example of preferred carbon sources are assimilable carbohydrates, such as glucose, maltose, mannitol, fructose or lactose, or an acetate such as sodium acetate, which can be used either alone or in suitable mixtures. Suitable nitrogen sources include, for example, amino acids, such as casamino acids, peptides and proteins and their degradation products, such as tryptone, peptone or meat extracts, furthermore yeast extract, malt extract, corn steep liquor, as well as ammonium salts, such as ammonium chloride, sulphate or nitrate which can be used either alone or in suitable mixtures. Inorganic salts which may be used include, for example, sulphates, chlorides, phosphates and carbonates of sodium, potassium, magnesium and calcium. Additionally, the nutrient medium may also contain growth promoting substances. Substances which promote growth include, for example, trace elements, such as iron, zinc, manganese and the like, or individual amino acids.

The monomeric form of the TGF-β-like protein can be produced by means of recombinant DNA technology or synthetically by methods well known in the art. The dimeric form is the mature, biologically active molecule consisting of two disulfide-linked polypeptide chains. This dimeric form can either be produced directly in an appropriate expression system well known in the art, e.g. CHO cells, or preferentially by expressing the monomer in a microbial host cell, preferentially in E. coli, and subjecting this monomer to refolding.

Thus, the invention relates to a process for the production of a dimeric, biologically active hybrid TGF-β protein, in which the monomeric form of a hybrid TGF-β protein, preferentially one of those hybrids specified hereinbefore, is produced by the steps of:

(a) culturing a microbial host comprising a nucleotide sequence encoding the hybrid TGF-β protein linked in the proper reading frame to an expression control sequence such that said protein is expressed,

(b) recovering the hybrid TGF-β protein in a denatured, monomeric, soluble form.

The monomeric hybrid TGF-β protein is recovered from the microbial host cells by methods well known in the art. These methods include lysis or mechanical disruption of the cells in order to release the desired protein, followed by the separation of the hybrid TGF-β protein from the host cell proteins, e.g. by precipitation and/or chromatographic means.

In cases where the monomeric hybrid TGF-β protein is produced in the microbial host cells as an insoluble aggregate (inclusion body) it has to be solubilized before being exposed to the refolding conditions. Accordingly, the present invention further relates to a process wherein the monomeric hybrid TGF-β protein is produced by the steps of:

(a) isolating the water-insoluble protein fraction containing the hybrid TGF-β protein from the host cells and

(b) solubilizing the hybrid TGF-β protein.

Solubilization and denaturation of the monomer is achieved by acidification of the crude protein suspension containing the monomeric hybrid TGF-β protein in the non-soluble form to a pH of about 1 to about 4, preferably to about 2.5, optionally in the presence of a reducing agent, such as DTT, or by the addition of chaotropic agents, preferably guanidine HCl or most preferably urea, in a concentration of about 4 to 9 M, basic pH or elevated temperatures as described before. The solubilized monomer can be purified from solubilizing chaotropes by dialysis and, if a precipitate occurs during dialysis, by additional centrifugation. The solubilized monomer is chromatographically purified and used for refolding to get the biologically active, dimeric product.

Refolding is performed using a refolding process known in the art (European Patent Application EP-A-0 433 225). In such a refolding process the in vitro refolding of monomeric, denatured TGF-β hybrid protein into the biologically active, disulfide-linked, dimeric form is achieved by mixing a solution of monomeric denatured TGF-β hybrid protein with a refolding buffer. Refolding buffer consists of a buffering salt, e.g. Tris/HCl, an additional salt, e.g. NaCl, optionally a chelating agent, e.g. EDTA, a sulfhydryl/disulfide redox system, e.g. glutathione in its reduced and oxidized form, respectively, and a solubilizing agent which permits folding of the monomeric TGF-β hybrid protein into the spatial conformation which is associated with the biological activity, while retaining said monomer, the folding intermediates and the dimer in solution.

Preferred solubilizing agents are the zwitterionic detergents 3-(3-chlolamidopropyl)dimethyl-ammonio-1-propansulfonate [CHAPS] or 3-(3-chlolamidopropyl)dimethyl-ammonio-2-hydroxy-1-propansulfonate [CHAPSO], or other detergents with similar solubilizing characteristics. Most preferred is CHAPS.

Preferred pH, temperature and concentrations of the chemicals contained in the refolding buffer are: buffering salt: about 10 mM to about 1 M, most preferably about 100 mM; additional salt: about 10 mM to about 2 M, most preferably about 1 M; chelating agent: about 0.1 to about 100 mM, most preferably about 2 mM; sulfhydryl/disulfide redox agents: about 0.1 to about 10 mM, with a molar ratio between about 100:1 and about 1:100, most preferably about 2.5 mM reduced and about 1 mM oxidized glutathione; solubilizing agent; about 10 to about 100 mM, most preferably about 30 mM; pH: about 7 to about 10, most preferably between about 8 and 9; temperature: about 0 °C and 37 °C, most preferably about 4°C.

After refolding, the biologically active dimer is purified in order to remove impurities, in particular, pyrogens or other endotoxins which might be present in the preparation after production of the recombinant protein in microbial host cells. Separation of the dimer is performed by chromatography such as sizing gel chromatography, hydrophobic interaction chromatography or ion exchange chromatography, e.g. on a Mono S column, and reverse phase HPLC.

The present invention further relates to dimeric biologically active hybrid TGF-β proteins when produced according to the process of the invention. These hybrid TGF-β proteins can be used in a variety of therapeutic modalities.

The present invention concerns further a pharmaceutical composition comprising an effective amount of a dimeric, biologically active hybrid TGF-β protein produced according to the invention, or a pharmaceutically acceptable salt thereof in dosage unit form.

Such composition is in the form of infusion solutions or preparations for parenteral, for example intramuscular or intravenous, oral, or especially for local, i.e. topical, administration, respectively. The solutions are preferably isotonic aqueous solutions or suspensions which can be prepared before use, for example from lyophilised preparations which contain the active ingredient alone or together with a pharmaceutically acceptable carrier. Solutions for parenteral use are usually aqueous solutions. They are prepared in conventional manner and may contain in addition to the active ingredient physiological saline, a stabilizer, such as human serum albumin, amino acids, such as arginine or glycine, and a carbohydrate, such as glucose, mannose, dextran or hydroxyethyl starch. The pH may be adjusted with a buffer, e.g. a phosphate, succinate or an amino acid to about 4.5 to 7. Usually the vials are filled with the solution and lyophilized for longer storage.

The compositions contain conventional adjuncts, for example preservatives, stabilisers, wetting agents and/or emulsifiers, solubilisers, salts for regulating the osmotic pressure and/or buffers. The present pharmaceutical compositions, which may, if desired, contain further pharmacologically valuable substances, are produced in a manner known per se, for example by means of conventional mixing, dissolving, lyophilising and/or sterilising processes, and contain from approximately 1 ng to 100 µg/g, especially from approximately 10 ng to 10 µg/g of preparation, and in the case of lyophilisates up to 100 %, of the active ingredient.

The hybrid TGF-β proteins are dual in character in that they on the one hand stimulate the proliferation of certain cell types, namely fibroblasts, and on the other hand inhibit the proliferation of other cell types, namely tumor cells and cells of the immune system.

The dimeric, biologically active hybrid TGF-β proteins produced according to the invention, optionally in the form of their salts, such as in particular non-toxic pharmaceutical acid addition salts, optionally in form of pharmaceutical formulations, are applied in an effective amount. By the form "effective amount" is intended an amount which exerts a significant healing, e.g. an amount which stimulates the desired cells to grow and which is not toxic to normal cells. This amount can be determined e.g. by in vitro growth experiments. Due to the dual character of hybrid TGF-β proteins, an "effective amount" is also such which to a significant extent inhibits the growth and proliferation of tumour cells and cells of the immune system. If human or veterinary use is intended, the amount has to be adjusted to the particular tissue to be treated, the mode of application, the severity of the disease, and the age and general condition of the patient to be treated. In general, the either single or daily dosages for adult humans will be in the range of about 0.01 to 20 µg for both the growth stimulating and the inhibiting effect.

The pharmaceutical composition of this invention have a clinical use in the treatment of animals, particularly mammals, more particularly human beings, and, in the case of wound healing, most particularly of old human beings.

The compositions of this invention promote cell migration and proliferation. Since wound healing involves both cell migration and cell proliferation patterns these in vitro findings become directly relevant to the in vivo wound healing process.

Prevention or treatment of bed sores (decubitus ulcers) is a preferred use since they frequently occur in hospital patients, particularly geriatric and wheel chair patients. In elderly people the wound healing process is slower and this group of patients tends to show a higher incidence of wounds (not only decubitus and diabetic ulcers, but trauma, burns and the like) that either heal slowly or do not heal at all.

Two types of application of the compositions of this invention are proposed for both veterinary and, in particular, human medicine.

The first, and preferred application is a topical one for the promotion of surface wound healing, particularly in elderly human beings where the wound healing processes are noticeably slower. There are no limitations as to the type of wound that may be treated, and these include (but are not limited to): Surface ulcers including decubital (bed sore), diabetic, dental, oral, varicose and haemophiliac surface ulcers; bums (especially second and third degree); surgical incisions (including those of dental and cosmetic surgery); accidental wounds (including incisions, penetrations, lacerations and other traumata) and therapeutically induced wounds (including those induced during radiotherapy). When applied topically, the compositions may be combined with other ingredients, such as adjuvants, carriers, solubilizing agents and any other known, or as yet unknown, secondary growth factor(s). There are no limitations as to the nature of these ingredients except that they must be pharmaceutically and physiologically acceptable for administration and must not degrade the activity, or render harmfully toxic, the active ingredients of the compositions. When the compositions of this invention are applied to surface ulcers, burns, surgical or accidental wounds, the compositions are preferably in the form of a powder, gel, ointment, salve or irrigant, or they may be impregnated into transdermal patches, plasters and bandages, preferably in a liquid or semi-liquid form, or they may be incorporated into a tooth paste or a gum or resin for chewing.

The second application is a systemic one for the healing of internal wounds either following surgery, or damage to the tissues of the inner organs where surgery is either impossible or is not required. Again, there are no limitations as to the type of tissue or wound to be treated and these include (but are not limited to) deep surgical incisions to the inner organs and tissues; bone and cartilage (after fracture); gastric, duodenal and other intestinal ulcers. When applied systemically, the compositions of the invention may be formulated as liquids, pills, tablets, lozenges for enteral administration, or in liquid form for parenteral injection. For the treatment of internal incisions following surgery, they may be in the form of an irrigant, preferably in combination with a physiologically acceptable saline solution. Again, the active ingredients of the compositions may be combined with other ingredients such as adjuvants, carriers, solubilizing agents and any other known, or as yet unknown, secondary growth factor(s). There are no limitations as to the nature of these ingredients except that they must be pharmaceutically and physiologically acceptable for administration and must not degrade the activity, or render harmfully toxic, the active ingredients of these compositions.

For healing the wounds, the amount of active ingredient to be applied has to be adjusted to the type, severity and location of the wound, and also to the age and general condition of the patient to be treated. In general a single or daily amount of from about 0. 1 $\mu$g to 20 $\mu$g of hybrid TGF-$\beta$ protein per 1 cm$^2$ of wound has already a significant healing effect. For internal use a higher amount should be applied depending on the mode of administration due to the dilution of the hybrid TGF-$\beta$ protein in the body fluids.

Further uses of the hybrid TGF-$\beta$ proteins produced according to the invention are in bone and tissue repair, treatment of cancer in mammals, as an anti-inflammatory or immuno-suppressive agent, as a growth regulator in mammalian cell cultures or as a bone marrow protective agent or mediator of cardioprotection.

The most preferred embodiments of the present invention are those described hereinafter in the Examples.

The following Examples are illustrating the present invention, however, are in no way intended to limit it.

Examples

Example 1: Construction of hybrid TGF-$\beta$ cDNAs

The sequences of the TGF-$\beta$1, TGF-$\beta$2 and TGF-$\beta$3 cDNA referred to hereinafter is depicted in the Sequence listing under SEQ ID NO. 1 to 3.

The various oligomers used for construction of the hybrid cDNAs are synthesized on an Applied Biosystems DNA Synthesizer and are all depicted in the Sequence listing.

Using Polymerase Chain Reaction (PCR) technique TGF-$\beta$1, TGF-$\beta$2 and TGF-$\beta$3 cDNA fragments are firstly amplified and then joined together in various twin combinations to produce the corresponding TGF-$\beta$1(44/45)$\beta$2, TGF-$\beta$2(44/45)$\beta$1, TGF-$\beta$1(44/45)$\beta$3, TGF-$\beta$3(44/45)$\beta$1, TGF-$\beta$2(44/45)$\beta$3 and TGF-$\beta$3(44/45)$\beta$2 hybrid cDNAs (see SEQ ID NO. 4 to 9)as well as TGF-$\beta$1(56/57)$\beta$2, TGF-$\beta$2(56/57)$\beta$1, TGF-$\beta$1(56/57)$\beta$3, TGF-$\beta$3(56/57)$\beta$1, TGF-$\beta$2(56/57)$\beta$3, TGF-$\beta$3(56/57)$\beta$2, TGF-$\beta$1(79/80)$\beta$2, TGF-$\beta$2(79/80)$\beta$1, TGF-$\beta$1(79/80)$\beta$3, TGF-$\beta$3(79/80)$\beta$1, TGF-$\beta$2(79/80)$\beta$3, TGF-$\beta$3(79/80)$\beta$2, TGF-$\beta$1(90/91)$\beta$2, TGF-$\beta$2(90/91)$\beta$1, TGF-$\beta$1(90/91)$\beta$3, TGF-$\beta$3(90/91)$\beta$1, TGF-$\beta$2(90/91)$\beta$3, TGF-$\beta$3(90/91)$\beta$2, TGF-$\beta$1(22/23)$\beta$2, TGF-$\beta$2(22/23)$\beta$1, TGF-$\beta$1(22/23)$\beta$3, TGF-$\beta$3(22/23)$\beta$1, TGF-$\beta$2(22/23)$\beta$3, TGF-$\beta$3(22/23)$\beta$2 as described hereinafter.

All polymerase chain reactions (PCR) are performed with 50ng of the corresponding TGF-$\beta$1, TGF-$\beta$2 or

TGF-β3 template. Amplification is performed in the presence of 2 x 2μg of the respective oligomers in a 100μl reaction mixture containing 10mM TRIS/HCl (pH8.35), 50mM KCl, 1.5mM MgCl2, 0.05% (w/v) NP40, 0.05% (w/v) Tween 20 and 200μM of each dATP, dGTP, dCTP and dTTP using 5 units Vent Polymerase (New England Biolabs). 30 rounds of amplification are performed under the following temperatures using a Perkin-Elmer Cetus Heating Block: 93 °C/0.1 minutes, 45 °C/0.2 minutes, 73 °C/1.5 minutes.

For the construction of all DNA molecules encoding any of the above-referenced TGF-β hybrid molecules, following oligomers are used which are corresponding to modified nucleotide sequences of the cDNAs (see SEQ ID NO. 1, 2 and 3) of

- TGF-β1: oligomers 1 and 2, SEQ ID NO. 10 and 11, respectively,
- TGF-β2: oligomers 3 and 4, SEQ ID NO. 12 and 13, respectively, and
- TGF-β3: oligomers 5 and 6, SEQ ID NO. 14 and 15, respectively.

These oligonucleotides represent the 5' (oligomers 1,3 and 5) and 3' (2,4 and 6) sequences which flank the regions coding for the mature forms (112 amino acids) of the respective protein. In addition, oligomers 7 to 36 (se SEQ ID Nos. 16 to 45) which correspond to nucleotide sequences within highly conserved coding region in the respective TGF-β1, TGF-β2 and TGF-β3 cDNA molecules are also synthesized.

For the construction of the DNA molecules encoding the TGF-β1(44/45)β2, TGF-β2(44/45)β1, TGF-β1(44/45)β3, TGF-β3(44/45)β1, TGF-β2(44/45)β3, and TGF-β3(44/45)β2, all having the hinge points between amino acids 44 and 45, oligomers 7, 8, 11, 12, 9 and 10, corresponding to SEQ ID NO. 16, 17, 20, 21, 18, and 19, respectively, are used in addition to oligomers 1 to 6.

Using oligomers 1 and 8, a 146 bp long fragment which includes the coding region of the first 44 amino acids at the N-terminus of the TGF-β1 mature form protein is amplified in a first PCR reaction from a TGF-β1 cDNA template. The second moiety of the hybrid TGF-β cDNA is likewise generated in a second PCR reaction using oligmers 7 and 4, resulting in the amplification of a 241bp long fragment from a TGF-β2 cDNA template which includes the coding region of the last 68 amino acids at the C-terminus of the TGF-β2 mature form protein. The PCR reactions are phenol/chlororform extracted and ethanol precipitated.The amplification products are then dissolved in 10mM TRIS/HCl(pH 7.5), 1 mM EDTA and gel filtrated over a A-15m Biogel column (Bio Rad). In a third PCR reaction, using oligomers 1 and 4, these amplification products are pooled to form the template for the generation of the hybrid TGF-β1(44/45)β2 cDNA.

Likewise, TGF-β1(44/45)β3, TGF-β2(44/45)β1, TGF-β2(44/45)β3, TGF-β3(44/45)β1 and TGF-β3(44/45)β2 hybrids are generated in three PCR reactions according to the method described above but using the oligomers and templates ("templ. cDNA") listed in the table below.

| TGF-ß Hybrid | 1st PCR React. | | 2nd PCR React. | | 3rd PCR React. | |
|---|---|---|---|---|---|---|
| | Oligomers | templ. cDNA | Oligomers | templ. cDNA | Oligomers | templ. cDNA |
| β1 (44/45) β2 | 1 & 8 | TGF-β1 | 7 & 4 | TGF-β2 | 1 & 4 | * |
| β1 (44/45) β3 | 1 & 8 | TGF-β1 | 7 & 6 | TGF-β3 | 1 & 6 | * |
| β2 (44/45) β1 | 3 & 12 | TGF-β2 | 11& 2 | TGF-β1 | 3 & 2 | * |
| β2 (44/45) β3 | 3 & 12 | TGF-β2 | 11& 6 | TGF-β3 | 3 & 6 | * |
| β3 (44/45) β1 | 5 & 10 | TGF-β3 | 9 & 2 | TGF-β1 | 5 & 2 | * |
| β3 (44/45) β2 | 5 & 10 | TGF-β3 | 9 & 4 | TGF-β2 | 5 & 4 | * |

* in each example, the reaction products from the 1st and 2nd PCR reactions form the DNA template for the 3rd PCR reaction.

For the construction of hybrids TGF-β1(22/23)β2, TGF-β2(22/23)β1, TGF-β1(22/23)β3, TGF-β3(22/23)β1, TGF-β2(22/23)β3, and TGF-β3(22/23)β2, all having the hinge point between amino acid 22 and 23, also oligomers 1 to 6 and additionally the following oligonucleotides are used which correspond to modified nucleotide sequences of

- TGF-β1: oligo 13 and 14 (SEQ ID NO. 22 and 23, respectively)
- TGF-β2: oligo 15 and 16 (SEQ ID NO. 24 and 25, respectively)
- TGF-β3: oligo 17 and 18 (SEQ ID NO. 26 and 27, respectively).

The hybrids are generated in three PCR reactions according to the method described above but using the oligomers and templates ("templ. cDNA") listed in the table below.

| TGF-ß Hybrid | 1st PCR React. | | 2nd PCR React. | | 3rd PCR React. | |
|---|---|---|---|---|---|---|
| | Oligomers | templ. cDNA | Oligomers | templ. cDNA | Oligomers | templ. cDNA |
| β1 (22/23) β2 | 1 & 14 | TGF-β1 | 13 & 4 | TGF-β2 | 1 & 4 | * |
| β1 (22/23) β3 | 1 & 14 | TGF-β1 | 13 & 6 | TGF-β3 | 1 & 6 | * |
| β2 (22/23) β1 | 3 & 16 | TGF-β2 | 15 & 2 | TGF-β1 | 3 & 2 | * |
| β2 (22/23) β3 | 3 & 16 | TGF-β2 | 15 & 6 | TGF-β3 | 3 & 6 | * |
| β3 (22/23) β1 | 5 & 18 | TGF-β3 | 17 & 2 | TGF-β1 | 5 & 2 | * |
| β3 (22/23) β2 | 5 & 18 | TGF-β3 | 17 & 4 | TGF-β2 | 5 & 4 | * |

* in each example, the reaction products from the 1st and 2nd PCR reactions form the DNA template for the 3rd PCR reaction.

For the construction of hybrids TGF-β1(56/57)β2, TGF-β2(56/57)β1, TGF-β1(56/57)β3, TGF-β3(56/57)β1, TGF-β2(56/57)β3, and TGF-β3(56/57)β2, all having the hinge point between amino acid 56 and 57, also oligomers 1 to 6 and additionally the following oligonucleotides are used which correspond to modified nucleotide sequences of
- TGF-β1: oligo 19 and 20 (SEQ ID NO. 28 and 29, respectively)
- TGF-β2: oligo 21 and 22 (SEQ ID NO. 30 and 31, respectively)
- TGF-β3: oligo 23 and 24 (SEQ ID NO. 32 and 33, respectively)

The hybrids are generated in three PCR reactions according to the method described above but using the oligomers and templates ("templ. cDNA") listed in the table below.

| TGF-ß Hybrid | 1st PCR React. | | 2nd PCR React. | | 3rd PCR React. | |
|---|---|---|---|---|---|---|
| | Oligomers | templ. cDNA | Oligomers | templ. cDNA | Oligomers | templ. cDNA |
| β1 (56/57) β2 | 1 & 20 | TGF-β1 | 19 & 4 | TGF-β2 | 1 & 4 | * |
| β1 (56/57) β3 | 1 & 20 | TGF-β1 | 19 & 6 | TGF-β3 | 1 & 6 | * |
| β2 (56/57) β1 | 3 & 22 | TGF-β2 | 21 & 2 | TGF-β1 | 3 & 2 | * |
| β2 (56/57) β3 | 3 & 22 | TGF-β2 | 21 & 6 | TGF-β3 | 3 & 6 | * |
| β3 (56/57) β1 | 5 & 24 | TGF-β3 | 23 & 2 | TGF-β1 | 5 & 2 | * |
| β3 (56/57) β2 | 5 & 24 | TGF-β3 | 23 & 4 | TGF-β2 | 5 & 4 | * |

* in each example, the reaction products from the 1st and 2nd PCR reactions form the DNA template for the 3rd PCR reaction.

For the construction of hybrids TGF-β1(79/80)β2, TGF-β2(79/80)β1, TGF-β1(79/80)β3, TGF-β3(79/80)β1, TGF-β2(79/80)β3, and TGF-β3(79/80)β2, all having the hinge point between amino acid 79 and 80, also oligomers 1 to 6 and additionally the following oligonucleotides are used which correspond to modified nucleotide sequences of

- TGF-β1: oligo 25 and 26 (SEQ ID NO. 34 and 35, respectively)
- TGF-β2: oligo 27 and 28 (SEQ ID NO. 36 and 37, respectively)
- TGF-β3: oligo 29 and 30 (SEQ ID NO. 38 and 39, respectively).

The hybrids are generated in three PCR reactions according to the method described above but using the oligomers and templates ("templ. cDNA") listed in the table below.

| TGF-ß Hybrid | 1st PCR React. | | 2nd PCR React. | | 3rd PCR React. | |
|---|---|---|---|---|---|---|
| | Oligomers | templ. cDNA | Oligomers | templ. cDNA | Oligomers | templ. cDNA |
| β1 (79/80) β2 | 1 & 26 | TGF-β1 | 25 & 4 | TGF-β2 | 1 & 4 | * |
| β1 (79/80) β3 | 1 & 26 | TGF-β1 | 25 & 6 | TGF-β3 | 1 & 6 | * |
| β2 (79/80) β1 | 3 & 28 | TGF-β2 | 27 & 2 | TGF-β1 | 3 & 2 | * |
| β2 (79/80) β3 | 3 & 28 | TGF-β2 | 27 & 6 | TGF-β3 | 3 & 6 | * |
| β3 (79/80) β1 | 5 & 30 | TGF-β3 | 29 & 2 | TGF-β1 | 5 & 2 | * |
| β3 (79/80) β2 | 5 & 30 | TGF-β3 | 29 & 4 | TGF-β2 | 5 & 4 | * |

* in each example, the reaction products from the 1st and 2nd PCR reactions form the DNA template for the 3rd PCR reaction.

For the construction of hybrids TGF-β1(90/91)β2, TGF-β2(90/91)β1, TGF-β1(90/91)β3, TGF-β3(90/91)β1, TGF-β2(90/91)β3, and TGF-β3(90/91)β2, all having the hinge point between amino acid 90 and 91, also oligomers 1 to 6 and additionally the following oligonucleotides are used which correspond to modified nucleotide sequences of

- TGF-β1: oligo 31 and 32 (SEQ ID NO. 40 and 41, respectively)
- TGF-β2: oligo 33 and 34 (SEQ ID NO. 42 and 43, respectively)
- TGF-β3: oligo 35 and 36 (SEQ ID NO. 44 and 45, respectively).

The hybrids are generated in three PCR reactions according to the method described above but using the oligomers and templates ("templ. cDNA") listed in the table below.

| TGF-ß Hybrid | 1st PCR React. | | 2nd PCR React. | | 3rd PCR React. | |
|---|---|---|---|---|---|---|
| | Oligo-mers | templ. cDNA | Oligo-mers | templ. cDNA | Oligo-mers | templ. cDNA |
| β1 (90/91) β2 | 1 & 32 | TGF-β1 | 31 & 4 | TGF-β2 | 1 & 4 | * |
| β1 (90/91) β3 | 1 & 32 | TGF-β1 | 31 & 6 | TGF-β3 | 1 & 6 | * |
| β2 (90/91) β1 | 3 & 34 | TGF-β2 | 33 & 2 | TGF-β1 | 3 & 2 | * |
| β2 (90/91) β3 | 3 & 34 | TGF-β2 | 33 & 6 | TGF-β3 | 3 & 6 | * |
| β3 (90/91) β1 | 5 & 36 | TGF-β3 | 35 & 2 | TGF-β1 | 5 & 2 | * |
| β3 (90/91) β2 | 5 & 36 | TGF-β3 | 35 & 4 | TGF-β2 | 5 & 4 | * |

* in each example, the reaction products from the 1st and 2nd PCR reactions form the DNA template for the 3rd PCR reaction.

In each case the third PCR reaction is phenol/chloroform extracted and ethanol precipitated. The precipitate is then digested to completion with NcoI and SalI following the recommendations of the supplier (Boehringer). The hybrid TGF-β cDNA molecule is purified by gel electrophoresis (Ultrapure BRL) using NA-45 DEAE paper (Schleicher and Schuell). The DNA is eluted from the paper in 50mM TRIS/HCl (pH7.5), 5mM EDTA, 1M NaCl and is then phenol/chloroform extracted and ethanol precipitated. The resulting hybrid TGF-β DNA pellets is washed with 70% ethanol, resuspended in 10mM TRIS/HCl (pH7.5), 1mM EDTA and then sub-cloned into plasmid pGEM-5zf(+)(Promega) via the NcoI and SalI sites.

The constructs resulting from the preparation of the DNA encoding the hybrids with the hinge point between amino acid 44 and 45 are designated as pGKM.D98(TGF-β1(44/45)β2) pGKM.D571(TGF-β2(44/45)β1), pGKM.D99(TGF-β1(44/45)β3), pGKM.D584(TGF-β3(44/45)β1), pGKM.D580(TGF-β2(44/45)β3) and pGKM.D619 (TGF-β3(44/45)β2) and are used to transform competent E.coli JM109 cells (see Example 2). Clones carrying the correct inserts encoding the hybrid TGF-β1(44/45)β2, TGF-β2(44/45)β1, TGF-β1(44/45)β3, TGF-β3(44/45)β1, TGF-β2(44/45)β3 and TGF-β3(44/45)β2 are designated as E.coli JM109 pGKM.D98(TGF-β1(44/45)β2), E.coli JM109 pGKM.D571(TGF-β2(44/45)β1), E.coli JM109 pGKM.D99(TGF-β1(44/45)β3), E.coli JM109 pGKM.D584(TGF-β3(44/45)β1), E.coli JM109 pGKM.D580(TGF-β2(44/45)β3) and E.coli JM109 pGKM.D619(TGF-β3(44/45)β2), respectively.

Likewise the constructs resulting from the preparation of the DNA encoding the hybrids with the hinge point between amino acids 56 and 57 are designated as pGKM.D98(TGF-β1(56/57)β2) pGKM.D571 (TGF-β2(56/57)β1), pGKM.D99(TGF-β1(56/57)β3), pGKM.D584(TGF-β3(56/57)β1), pGKM.D580(TGF-β2(56/57)β3), and pGKM.D619(TGF-β3(56/57)β2) and are used to transform competent E.coli JM109 cells (see Example 2). Clones carrying the correct inserts encoding the hybrid TGF-β1(56/57)β2, TGF-β2(56/57)β1, TGF-β1(56/57)β3, TGF-β3(56/57)β1, TGF-β2(56/57)β3 and TGF-β3(56/57)β2 are designated as E.coli JM109 pGKM.D98(TGF-β1(56/57)β2), E.coli JM109 pGKM.D571(TGF-β2(56/57)β1), E.coli JM109 pGKM.D99(TGF-β1(56/57)β3),E.coli JM109 pGKM.D584(TGF-β3(56/57)β1), E.coli JM109 pGKM.D580(TGF-β2(56/57)β3), and E.coli JM109 pGKM.D619(TGF-β3(56/57)β2), respectively.

Likewise the constructs resulting from the preparation of the DNA encoding the hybrids with the hinge point between amino acids 79 and 80 are designated as pGKM.D98(TGF-β1(79/80)β2) pGKM.D571(TGF-β2(79/80)β1), pGKM.D99(TGF-β1(79/80)β3), pGKM.D584(TGF-β3(79/80)β1), pGKM.D580(TGF-β2(79/80)β3), and pGKM.D619(TGF-β3(79/80)β2) and are used to transform competent E.coli JM109 cells (see Example 2). Clones carrying the correct inserts encoding the hybrid TGF-β1(79/80)β2, TGF-β2(79/80)β1, TGF-β1(79/80)β3, TGF-β3(79/80)β1, TGF-β2(79/80)β3 and TGF-β3(79/80)β2 are designated as E.coli JM109 pGKM.D98(TGF-β1(79/80)β2), E.coli JM109 pGKM.D571(TGF-β2(79/80)β1), E.coli JM109 pGKM.D99(TGF-β1(79/80)β3),E.coli JM109 pGKM.D584(TGF-β3(79/80)β1), E.coli JM109 pGKM.D580(TGF-β2(79/80)β3), and E.coli JM109 pGKM.D619(TGF-β3(79/80)β2), respectively.

Likewise, the constructs resulting from the preparation of the DNA encoding the hybrids with the hinge point between amino acids 90 and 91 are designated as pGKM.D98(TGF-β1(90/91)β2) pGKM.D571 (TGF-β2(90/91)β1), pGKM.D99(TGF-β1(90/91)β3), pGKM.D584(TGF-β3(90/91)β1), pGKM.D580(TGF-β2(90/91)β3) and pGKM.D619(TGF-β3(90/91)β2) and are used to transform competent E.coli JM109 cells (see Example 2). Clones carrying the correct inserts encoding the hybrid TGF-β1(90/91)β2, TGF-β2(90/91)β1, TGF-β1(90/91)β3, TGF-β3(90/91)β1, TGF-β2(90/91)β3 and TGF-β3(90/91)β2 are designated as E.coli JM109 pGKM.D98(TGF-β1(90/91)β2), E.coli JM109 pGKM.D571(TGF-β2(90/91)β1), E.coli JM109 pGKM.D99(TGF-β1(90/91)β3), E.coli JM109 pGKM.D584(TGF-β3(90/91)β1), E.coli JM109 pGKM.D580(TGF-β2(90/91)β3) and E.coli JM109 pGKM.D619(TGF-β3(90/91)β2), respectively.

Likewise, the constructs resulting from the preparation of the DNA encoding the hybrids with the hinge point between amino acid 22 and 23 are designated as pGKM.D98(TGF-β1(22/23)β2) pGKM.D571 (TGF-β2(22/23)β1), pGKM.D99(TGF-β1(22/23)β3), pGKM.D584(TGF-β3(22/23)β1), pGKM.D580(TGF-β2(22/23)β3) and pGKM.D619(TGF-β3(22/23)β2) and are used to transform competent E.coli JM109 cells (see Example 2). Clones carrying the correct inserts encoding the hybrid TGF-β1(22/23)β2, TGF-β2(22/23)β1, TGF-β1(22/23)β3, TGF-β3(22/23)β1, TGF-β2(22/23)β3 and TGF-β3(22/23)β2 are designated as E.coli JM109 pGKM.D98(TGF-β1(22/23)β2), E.coli JM109 pGKM.D571(TGF-β2(22/23)β1), E.coli JM109 pGKM.D99(TGF-β1(22/23)β3), E.coli JM109 pGKM.D584(TGF-β3(22/23)β1), E.coli JM109 pGKM.D580(TGF-β2(22/23)β3) and E.coli JM109 pGKM.D619(TGF-β3(22/23)β2), respectively.

## Example 2: Sequencing of hybrid TGF-β cDNAs

Identities of the various hybrid TGF-β cDNAs are confirmed by double-stranded sequencing according to the Sanger-method [PNAS 74:5463(1977)] using oligomers 7,8,9,10,11, and 12, standard SP6 and T7 primers (Promega) and a Sequenase kit (U.S.Biochemicals). The nucleotide sequence covering the 112 amino acids of the mature TGF-β1(44/45)β2, TGF-β1(44/45)β3, TGF-β2(44/45)β1, TGF-β2(44/45)β3, TGF-β3(44/45)β1 and TGF-β3(44/45)β2 hybrids, plus an additional methionine residue at the N-terminus, are depicted under SEQ ID NO.4,5,6,7,8 and 9, respectively (start codon ATG not shown).

## Example 3: Expression of hybrid TGF-βs in E.coli

### 3A.General Methods

#### Bacterial strain (E. coli K12):

LC 137: htpR_{am}, lon_{R9}, lac_{am}, mal_{am}, trp_{am}, pho_{am}, rspL, tsx::Tn10, supC_{ts} (Goff, S.A. et al. (1984) PNAS 81, 6647-6651).

#### Plasmids:

pPLMu:    (Buell, G. et al. (1985) Nucleic Acids Res. 13, 1923-1938). This plasmid carries the bacteriophage λ P_L promoter with the phage Mu ner gene ribosome binding site (Van Leerdam, E. et al. (1982) Virology 123, 19-28).

pcl_{857}:    Plasmid encoding a thermolabile λCl_{857} repressor and conferring resistance to kanamycin (Remault, E. et al. (1983) Gene 22, 103-113).

#### SDS pel-electrophoresis:

SDS polyacrylamide gel-electrophoresis (SDS-PAGE) and protein staining is done as described previously (Laemmli, U.K. (1970) Nature 227, 680-685) using the Miniprotean II cell from BIORAD) and 1 mm thick 18 % polyacrylamide gels.

#### Heat induction:

7 ml of LB-Medium (Maniatis et al. (1982), Molecular Cloning, Cold Spring Harbor Laboratory, New York) in a 20 ml culture tube containing 40 μg of each ampicillin and kanamycin (LB/amp/kan) are inoculated with a single colony and incubated with shaking overnight at 30°C. 5 ml of this overnight culture are added to 15 ml of LB/amp/kan in a 100 ml Erlenmeyer flask. This flask is transferred to a 42°C waterbath shaker. A 2 ml sample is taken before transfer (non-inducing conditions) and 1 ml samples at 1 hour intervals after the transfer (inducing conditions). Cells are pelleted by centrifugation (5 min, 10.000 rpm in an Eppendorf centrifuge) and the supernatant is discarded. The pellet is resuspended in 100 μl of sample buffer for SDS-PAGE and heated for 10 min at 95°C. 5 μl aliquots are loaded for SDS-PAGE.

Preparation of competent cells:

Competent E. coli cells are prepared by the conventional calcium chloride procedure as described in Maniatis et al. (1982), Molecular Cloning, Cold Spring Harbor Laboratory, New York. Cells carrying plasmid pcl$_{857}$ are grown at 30°C.

### 3B. Construction of expression vectors and expression of hybrid TGF-βs

E.coli JM109 is transformed with the pGEM-5zf(+) vectors which contain the corresponding TGF-β1(44/45)β2, TGF-β2(44/45)β1, TGF-β1(44/45)β3, TGF-β3(44/45)β1, TGF-β2(44/45)β3 and TGF-β3(44/45)β2 hybrid DNAs. The E.coli cells are grown in LB medium and plasmid DNA is prepared. In each case 5μg of plasmid hybrid TGF-β DNA are cut to completion in 50μl restriction buffer with NcoI and SalI following the recommendations of the supplier (Boehringer). The DNA is precipitated by addition of 5μl 3M sodium acetate, 100mM MgCl2, 5mM EDTA, and 150μl Ethanol. After incubation at -70 °C for 15min the DNA is pelleted by centrifugation at 13.000g for 15 min in a SS-34 rotor in a Sorvall centrifuge. The supernatant is discarded and the pellet is resuspended in 80μl 0.089 M TRIS-Borate, 0.089 M Boric Acid and 0.002 M EDTA (TBE Buffer) containing 0.25% Bromphenol Blue and 0.25% Xylene Cyanol. 4 x 20μl samples are electrophoresed through a 1 % Agarose gel in TBE Buffer containing 0.5 μg/ml Ethidium Bromide at 50 volts until the Bromphenol Blue marker reaches the bottom of the 10 cm long and 0.8 cm-thick gel. The DNA fragments coding for the mature TGF-β1(44/45)β2, TGF-β2(44/45)β1, TGF-β1(44/45)β3, TGF-β3(44/45)β1, TGF-β2(44/45)β3 and TGF-β3(44/45)β2 hybrids respectively, are visualised under short wave U.V.light, cut out with a razor blade and electroeluted from a gel piece in a Schleicher and Schuell Biotrap Apparatus applying 200 milliamps for 1.5 h. The eluted DNA fragments are precipitated (see above) and resuspended in 20μl TE.

5μl of plasmid pPLMu are linearized by digestion with NcoI and SalI and gel purified as described above for the fragment DNAs. 100ng of the linearized and purified pPLMu vector DNA and 3x the molar equivalent of the respective purified fragment DNA are incubated at 4 °C for 15 h in 20 μl ligation buffer (70 mM TRIS-HCl, pH7.5, 10mM MgCl2, 5mM DTT, 0.1mM Adenosine-triphosphate) containing 1 unit of DNA ligase (Boehringer).

10μl of the ligation mixture are added to 200μl of cold (4 °C) competent E.coli LC137 cells carrying plasmid pcl857. After 30 min the cells are heat shocked by incubation for 1.5 min in a 42 °C water bath. 2ml of LB medium are added and the culture is shaken for 60 min at 30 °C. 200μl aliquots are plated on LB plates containing Ampicillin and Kanamycin and incubated for 22 h at 30 °C. Single colonies are cultivated and plasmid DNA is analysed. Subcloning of the DNA fragments coding for TGF-β1(44/45)β2, TGF-β2(44/45)β1, TGF-β1(44/45)β3, TGF-β3(44/45)β1, TGF-β2(44/45)β3 and TGF-β3(44/45)β2 in pPLMu results in plasmids pPLMu.TGF-β1(44/45)β2, pPLMu.TGF-β2(44/45)β1, pPLMu.TGF-β1(44/45)β3, pPLMu.TGF-β3(44/45)β1, pPLMu.TGF-β2(44/45)β3 and pPLMu.TGF-β3(44/45)β2, respectively. Clones containing the above constructs are referred to as E.coliLC137/pPLMu.TGF-β1(44/45)β2, E.coliLC137/pPLMu.TGF-β2(44/45)β1, E.coliLC137/pPLMu.TGF-β1(44/45)β3, E.coliLC137/pPLMu.TGF-β3(44/45)β1, E.coliLC137/pPLMu.TGF-β2(44/45)β3 and E.coliLC137/pPLMu.TGF-β3(44/45)β2, respectively.

Likewise, DNA fragments coding for TGF-β1(56/57)β2, TGF-β2(56/57)β1, TGF-β1(56/57)β3, TGF-β3(56/57)β1, TGF-β2(56/57)β3 and TGF-β3(56/57)β2 are subcloned in pPLMu to construct plasmids pPLMu.TGF-β1(56/57)β2, pPLMu.TGF-β2(56/57)β1, pPLMu.TGF-β1(56/57)β3, pPLMu.TGF-β3(56/57)β1, pPLMu.TGF-β2(56/57)β3 and pPLMu.TGF-β3(56/57)β2 and transformed hosts E.coliLC137/pPLMu.TGF-β1(56/57)β2, E.coliLC137/pPLMu.TGF-β2(56/57)β1, E.coliLC137/pPLMu.TGF-β1(56/57)β3, E.coliLC137/pPLMu.TGF-β3(56/57)β1, E.coliLC137/pPLMu.TGF-β2(56/57)β3 and E.coliLC137/pPLMu.TGF-β3(56/57)β2 are prepared.

Likewise, DNA fragments coding for TGF-β1(79/80)β2, TGF-β2(79/80)β1, TGF-β1(79/80)β3, TGF-β3(79/80)β1, TGF-β2(79/80)β3 and TGF-β3(79/80)β2 are subcloned in pPLMu to construct plasmids pPLMu.TGF-β1(79/80)β2, pPLMu.TGF-β2(79/80)β1, pPLMu.TGF-β1(79/80)β3, pPLMu.TGF-β3(79/80)β1, pPLMu.TGF-β2(79/80)β3 and pPLMu.TGF-β3(79/80)β2 and transformed hosts E.coliLC137/pPLMu.TGF-β1(79/80)β2, E.coliLC137/pPLMu.TGF-β2(79/80)β1, E.coliLC137/pPLMu.TGF-β1(79/80)β3, E.coliLC137/pPLMu.TGF-β3(79/80)β1, E.coliLC137/pPLMu.TGF-β2(79/80)β3 and E.coliLC137/pPLMu.TGF-β3(79/80)β2 are prepared.

Likewise, DNA fragments coding for TGF-β1(90/91)β2, TGF-β2(90/91)β1, TGF-β1(90/91)β3, TGF-β3(90/91)β1, TGF-β2(90/91)β3 and TGF-β3(90/91)β2 are subcloned in pPLMu to construct plasmids pPLMu.TGF-β1(90/91)β2, pPLMu.TGF-β2(90/91)β1, pPLMu.TGF-β1(90/91)β3, pPLMu.TGF-β3(90/91)β1, pPLMu.TGF-β2(90/91)β3 and pPLMu.TGF-β3(90/91)β2 and transformed hosts E.coliLC137/pPLMu.TGF-β1(90/91)β2, E.coliLC137/pPLMu.TGF-β2(90/91)β1, E.coliLC137/pPLMu.TGF-β1(90/91)β3, E.coliLC137/pPLMu.TGF-β3(90/91)β1, E.coliLC137/pPLMu.TGF-β2(90/91)β3 and E.coliLC137/pPLMu.TGF-β3(90/91)β2 are prepared.

Likewise, DNA fragments coding for TGF-β1(22/23)β2, TGF-β2(22/23)β1, TGF-β1(22/23)β3, TGF-β3(22/23)β1, TGF-β2(22/23)β3 and TGF-β3(22/23)β2 are subcloned in pPLMu to construct plasmids pPLMu.TGF-β1(22/23)β2, pPLMu.TGF-β2(22/23)β1, pPLMu.TGF-β1(22/23)β3, pPLMu.TGF-β3(22/23)β1, pPLMu.TGF-β2(22/23)β3 and pPLMu.TGF-β3(22/23)β2 and transformed hosts E.coliLC137/pPLMu.TGF-β1(22/23)β2, E.coliLC137/pPLMu.TGF-β2(22/23)β1, E.coliLC137/pPLMu.TGF-β1(22/23)β3, E.coliLC137/pPLMu.TGF-β3(22/23)β1, E.coliLC137/pPLMu.TGF-β2(22/23)β3 and E.coliLC137/pPLMu.TGF-β3(22/23)β2 are prepared.

E.coliLC137/pPLMu.TGF-β1(44/45)β2, E.coliLC137/pPLMu.TGF-β2(44/45)β1, E.coliLC137/pPLMu.TGF-β1(44/45)β3, E.coliLC137/pPLMu.TGF-β3(44/45)β1, E.coliLC137/pPLMu.TGF-β2(44/45)β3 and E.coliLC-137/pPLMu.TGF-β3(44/45)β2 are heat induced (see example 3.A) and the expressed proteins are analysed by SDS-PAGE. TGF-β1(44/45)β2, TGF-β2(44/45)β1, TGF-β1(44/45)β3, TGF-β3(44/45)β1, TGF-β2(44/45)β3 and TGF-β3(44/45)β2 all appear as heat induced proteins 2 h after heat induction migrating with an apparent molecular weight of approximately 12.000 D.

Likewise, E.coliLC137/pPLMu.TGF-β1(56/57)β2, E.coliLC137/pPLMu.TGF-β2(56/57)β1, E.coliLC137/pPLMu.TGF-β1(56/57)β3, E.coliLC137/pPLMu.TGF-β3(56/57)β1, E.coliLC137/pPLMu.TGF-β2(56/57)β3 and E.coliLC137/pPLMu.TGF-β3(56/57)β2 are heat induced (see example 3.A) and the expressed proteins are analysed by SDS-PAGE. TGF-β1(56/57)β2, TGF-β2(56/57)β1, TGF-β1(56/57)β3, TGF-β3(56/57)β1, TGF-β2(56/57)β3 and TGF-β3(56/57)β2 all appear as heat induced proteins 2 h after heat induction migrating with an apparent molecular weight of approximately 12.000 D.

Likewise, E.coliLC137/pPLMu.TGF-β1(79/80)β2, E.coliLC137/pPLMu.TGF-β2(79/80)β1, E.coliLC137/pPLMu.TGF-β1(79/80)β3, E.coliLC137/pPLMu.TGF-β3(79/80)β1, E.coliLC137/pPLMu.TGF-β2(79/80)β3 and E.coliLC137/pPLMu.TGF-β3(79/80)β2 are heat induced (see example 3.A) and the expressed proteins are analysed by SDS-PAGE. TGF-β1(79/80)β2, TGF-β2(79/80)β1, TGF-β1(79/80)β3, TGF-β3(79/80)β1, TGF-β2(79/80)β3 and TGF-β3(79/80)β2 all appear as heat induced proteins 2 h after heat induction migrating with an apparent molecular weight of approximately 12.000 D.

Likewise, E.coliLC137/pPLMu.TGF-β1(90/91)β2, E.coliLC137/pPLMu.TGF-β2(90/91)β1, E.coliLC137/pPLMu.TGF-β1(90/91)β3, E.coliLC137/pPLMu.TGF-β3(90/91)β1, E.coliLC137/pPLMu.TGF-β2(90/91)β3 and E.coliLC137/pPLMu.TGF-β3(90/91)β2 are heat induced (see example 3.A) and the expressed proteins are analysed by SDS-PAGE. TGF-β1(90/91)β2, TGF-β2(90/91)β1, TGF-β1(90/91)β3, TGF-β3(90/91)β1, TGF-β2(90/91)β3 and TGF-β3(90/91)β2 all appear as heat induced proteins 2 h after heat induction migrating with an apparent molecular weight of approximately 12.000 D.

Likewise, E. coli LC137/pPLMu.TGF-β1(22/23)β2, E.coliLC137/pPLMu.TGF-β2(22/23)β1, E.coliLC137/pPLMu.TGF-β1(22/23)β3, E.coliLC137/pPLMu.TGF-β3(22/23)β1, E.coliLC137/pPLMu.TGF-β2(22/23)β3 and E.coliLC137/pPLMu.TGF-β3(22/23)β2 are heat induced (see example 3.A) and the expressed proteins are analysed by SDS-PAGE. TGF-β1(22/23)β2, TGF-β2(22/23)β1, TGF-β1(22/23)β3, TGF-β3(22/23)β1, TGF-β2(22/23)β3 and TGF-β3(22/23)β2 all appear as heat induced proteins 2 h after heat induction migrating with an apparent molecular weight of approximately 12.000 D.

## 3C. Fermentation of Transformants

Overnight cultures of E.coliLC137/pPLMu.TGF-β1(44/45)β2, E.coliLC137/pPLMu.TGF-β2(44/45)β1, E.coliLC137/pPLMu.TGF-β1(44/45)β3, E.coliLC137/pPLMu.TGF-β3(44/45)β1, E.coliLC137/pPLMu.TGF-β2(44/45)β3 and E.coliLC137/pPLMu.TGF-β3(44/45)β2 in 2 l Erlenmeyer flasks containing 750 ml of LB medium with 40 mg/l of Ampicillin and Kanamycin are grown at 30 °C. 300 ml of the overnight cultures are added to 750 ml of LB medium containing antibiotics as mentioned above in 2 l Erlenmeyer flasks and heated to 42 °C by shaking for approximately 3.5 min in a 65 °C water bath. The flasks are then transferred to a 42 °C shaker and incubated for 3 h. The flasks are cooled down to 12 °C in an ice water bath and the cells are collected after centrifugation for 10 min at 8.000 rpm in a GSA rotor (Sorvall).

Likewise, hybrid proteins are produced by means of E.coliLC137/pPLMu.TGF-β1(56/57)β2, E.coliLC-137/pPLMu.TGF-β2(56/57)β1, E.coliLC137/pPLMu.TGF-β1(56/57)β3, E.coliLC137/pPLMu.TGF-β3(56/57)β1, E.coliLC137/pPLMu.TGF-β2(56/57)β3, and E.coliLC137/pPLMu.TGF-β3(56/57)β2.

Likewise, hybrid proteins are produced by means of E.coliLC137/pPLMu.TGF-β1(79/80)β2, E.coliLC-137/pPLMu.TGF-β2(79/80)β1, E.coliLC137/pPLMu.TGF-β1(79/80)β3, E.coliLC137/pPLMu.TGF-β3(79/80)β1, E.coliLC137/pPLMu.TGF-β2(79/80)β3, and E.coliLC137/pPLMu.TGF-β3(79/80)β2.

Likewise, hybrid proteins are produced by means of E.coliLC137/pPLMu.TGF-β1(90/91)β2, E.coliLC-137/pPLMu.TGF-β2(90/91)β1, E.coliLC137/pPLMu.TGF-β1(90/91)β3, E.coliLC137/pPLMu.TGF-β3(90/91)β1, E.coliLC137/pPLMu.TGF-β2(90/91)β3, and E.coliLC137/pPLMu.TGF-β3(90/91)β2.

Likewise, hybrid proteins are produced by means of E.coliLC137/pPLMu.TGF-β1(22/23)β2, E.coliLC-137/pPLMu.TGF-β2(22/23)β1, E.coliLC137/pPLMu.TGF-β1(22/23)β3, E.coliLC137/pPLMu.TGF-β3(22/23)β1, E.coliLC137/pPLMu.TGF-β2(22/23)β3, and E.coliLC137/pPLMu.TGF-β3(22/23)β2

## Example 4: Expression of TGF-β1/β2 TGF-β2/β1, TGF-β1/β3, TGF-β3/β1, TGF-β2/β3 and TGF-β3/β2 in Saccharomyces cerevisae

The coding sequences of mature TGF-β1(44/45)β2, TGF-β2(44/45)β1, TGF-β1(44/45)β3, TGF-β3(44/45)β1, TGF-β2(44/45)β3 and TGF-β3(44/45)β2 are expressed in Saccharomyces cerevisae under the control of the inducible promoter of the yeast acid phosphatase (PH05). They are obtained from plasmids pPLMu.TGF-β1(44/45)β2, pPLMu.TGF-β2(44/45)β1, pPLMu.TGF-β1(44/45)β3, pPLMu.TGF-β3(44/45)β1, pPLMu.TGF-β2(44/45)β3 and pPLMu.TGF-β3(44/45)β2, respectively.

The expression vectors are constructed in two steps:

A. construction of plasmid pJDB207/PH05-RIT-12,

B. construction of plasmids pJDB207R/PH05-TGF-β1(44/45)β2, pJDB207R/PH05-TGF-β2(44/45)β1, pJDB207R/PH05-TGF-β1(44/45)β3, pJDB207R/PH05-TGF-β3(44/45)β1, pJDB207R/PH05-TGF-β2(44/45)β3 and pJDB207R/PH05-TGF-β3(44/45)β2, where A) provides the yeast vector and the PH05 transcriptional terminator and B) provides the expression cassettes with an insert coding for mature TGF-β1(44/45)β2, TGF-β2(44/45)β1, TGF-β1(44/45)β3, TGF-β3(44/45)β1, TGF-β2(44/45)β3 and TGF-β3(44/45)β2, respectively, under the control of the PH05 promoter.

### A. Construction of plasmid pJDB207/PH05-RIT-12

Plasmid p31R/SS-TPAΔ2 (DSM 4295) is digested with restriction endonucleases EcoRI and XhoI. The 4.2 kb vector fragment is isolated by preparative agarose gel electrophoresis.

The four oligodeoxyribonucleotides I-1 (see SEQ ID No. 46), I-2 (SEQ ID No. 47), I-3 (SEQ ID No. 48) and I-4 (SEQ ID No. 49) are synthesized by a DNA synthesizer (model 380B Applied Biosystem) and purified on a 12% polyacrylamide gel containing 8 M urea. A solution of 10 pmoles of each of the four oligodeoxyribonucleotides I-1, I-2, I-3 and I-4 in 10 μl of 0.5 M Tris-HCl pH8 is incubated at 95 °C for 5 min on a water bath. The water bath is slowly cooled to 30 °C over a period of 5 h in order to anneal the complemetary oligonucleotides. To this annealed mixture is added 2 μl each of 0.1 M $MgCl_2$, 0.1 M NaCl, 30 mM DTT, 4 mM ATP and 8 U of polynucleotide kinase (Boehringer). Kination is carried out at 37 °C for 1 h.

The annealed, kinased oligonucleotides and 60 pmoles of the 4.2 kb EcoRI-XhoI vector fragment of p31R/SS-TPAΔ2 are ligated with T4 DNA ligase. The ligation mixture is used to transform competent E. coli HB101 cells.

Amp$^R$ colonies are picked, plasmid DNA is prepared, digested with EcoRI and XhoI, radiolabelled at the EcoRI end and analysed on a 6% polyacylamide gel containing 8M urea using HaeIII cut radiolabelled pBR322 as marker. One of the clones with correct band size is grown in LB, the plasmid is isolated and referred to as p31 RIT-12.

Plasmid p31 RIT-12 is linearized with restriction endonuclease SalI. Partial HindIII digestion in the presence of ethidiumbromide results in a 1 kb SalI/HindIII fragment comprising the 276 bp SalI/BamHI pBR322 sequence, the 534 bp promoter of the yeast acid phosphatase PH05, the yeast invertase signal sequence (coding for 19 amino acids) and the PH05 transcriptional terminator.

The 1 kb SalI/HindIII fragment of p31RIT-12 is cloned in to the yeast-E.coli shuttle vector pJDB207 (DSM 6782), which had been cut with SalI and HindIII. The resulting plasmid containing the 1 kb insert is referred to as pJDB207/PH05-RIT-12.

### B. Construction of plasmid pJDB207R/PH05-TGF-β1(44/45)β2

Plasmid pPLMu.TGF-β1(44/45)β2 is cut with NcoI. The sticky ends are filled in a reaction with Klenow DNA polymerase. EcoRI linker (5′-CCGGAATTCCGG; Biolabs) are added and the mixture is ligated. The resulting circular plasmid is referred to as pGKMA668 (TGF-β1(44/45)β2) and is cut with EcoRI and SalI. A 0.4 kb EcoRI/SalI fragment is isolated from an agarose gel, purified and resuspended in sterile water at a concentration of 25 μg/ml. The fragment contains the mature coding sequence of TGF-β1(44/45)β2 with an ATG in frame to codon GCT which defines amino acid Ala 1 of mature TGF-β1(44/45)β2.

The PH05 promoter is isolated from plasmid p31RIT 12 (see above) on a 534 bp BamHI/EcoRI fragment. Plasmid pJDB207/PH05-RIT-12 is cut with BamHI and XhoI. The large, 6.8 kb BamHI/XhoI fragment is isolated.

The PH05 transcriptional terminator remains on the fragment. The BamHI/EcoRI PH05 promoter fragment, the EcoRI/SalI fragment coding for TGF-β2, and the BamHI/XhoI vector fragment are ligated. One correct clone with the TGF-β2 gene under the control of the PH05 promoter cloned in an anticlockwise orientation into pJDB207 is referred to as pJDB207R/PH05-TGF-β1(44/45)β2.

In an analogous manner, mature TGF-β2(44/45)β1, TGF-β1(44/45)β3, TGF-β3(44/45)β1, TGF-β2(44/45)β3 and TGF-β3(44/45)β2 are expressed in S. cerevisiae. Plasmids containing the coding sequences of TGF-β1 and TGF-β3 are specified hereinbefore. After digestion of these plasmids with NcoI, addition of EcoRI linkers and ligation, the resulting circular plasmids are cut with EcoRI and SalI. The EcoRI/SalI fragments are cloned into pJDB207 as described above. The resulting plasmids are referred to as pJDB207R/PH05-TGF-β2(44/45)β1, pJDB207R/PH05-TGF-β1(44/45)β3, pJDB207R/PH05-TGF-β3(44/45)β1, pJDB207R/PH05-TGF-β2(44/45)β3 and pJDB207R/PH05-TGF-β3(44/45)β2.

In an analogous manner the expression vectors pJDB207R/PH05-TGF-β1(56/57)β2, pJDB207R/PH05-TGF-β2(56/57)β1, pJDB207R/PH05-TGF-β1(56/57)β3, pJDB207R/PH05-TGF-β3(56/57)β1, pJDB207R/PH05-TGF-β2(56/57)β3, and pJDB207R/PH05-TGF-β3(56/57)β2 are produced from pPLMu.TGF-β1(56/57)β2, pPLMu.TGF-β2(56/57)β1, pPLMu.TGF-β1(56/57)β3, pPLMu.TGF-β3(56/57)β1, pPLMu.TGF-β2(56/57)β3, and pPLMu.TGF-β3(56/57)β2 respectively, in order to express the coding sequences of mature TGF-β1(56/57)β2, TGFββ2(56/57)β1, TGF-β1(56/57)β3, TGF-β3(56/57)β1, TGF-β2(56/57)β3 and TGF-β3(56/57)β2.

In an analogous manner the expression vectors pJDB207R/PH05-TGF-β1(79/80)β2, pJDB207R/PH05-TGF-β2(79/80)β1, pJDB207R/PH05-TGF-β1(79/80)β3, pJDB207R/PH05-TGF-β3(79/80)β1, pJDB207R/PH05-TGF-β2(79/80)β3 and pJDB207R/PH05-TGF-β3(79/80)β2 are produced from pPLMu.TGF-β1(79/80)β2, pPLMu.TGF-β2(79/80)β1, pPLMu.TGF-β1(79/80)β3, pPLMu.TGF-β3(79/80)β1, pPLMu.TGF-β2(79/80)β3 and pPLMu.TGF-β3(79/80)β2, respectively, in order to express the coding sequences of mature TGF-β1(79/80)β2, TGFββ2(79/80)β1, TGF-β1(79/80)β3, TGF-β3(79/80)β1, TGF-β2(79/80)β3 and TGF-β3(79/80)β2.

In an analogous manner the expression vectors pJDB207R/PH05-TGF-β1(90/91)β2, pJDB207R/PH05-TGF-β2(90/91)β1, pJDB207R/PH05-TGF-β1(90/91)β3, pJDB207R/PH05-TGF-β3(90/91)β1, pJDB207R/PH05-TGF-β2(90/91)β3 and pJDB207R/PH05-TGF-β3(90/91)β2 are produced from pPLMu.TGF-β1(90/91)β2, pPLMu.TGF-β2(90/91)β1, pPLMu.TGF-β1(90/91)β3, pPLMu.TGF-β3(90/91)β1, pPLMu.TGF-β2(90/91)β3 and pPLMu.TGF-β3(90/91)β2, respectively, in order to express the coding sequences of mature TGF-β1(90/91)β2, TGF-β2(90/91)β1, TGF-β1(90/91)β3, TGF-β3(90/91)β1, TGF-β2(90/91)β3 and TGF-β3(90/91)β2.

In an analogous manner the expression vectors pJDB207R/PH05-TGF-β1(22/23)β2, pJDB207R/PH05-TGF-β2(22/23)β1, pJDB207R/PH05-TGF-β1(22/23)β3, pJDB207R/PH05-TGF-β3(22/23)β1, pJDB207R/PH05-TGF-β2(22/23)β3 and pJDB207R/PH05-TGF-β3(22/23)β2 are produced from pPLMu.TGF-β1(22/23)β2, pPLMu.TGF-β2(22/23)β1, pPLMu.TGF-β1(22/23)β3, pPLMu.TGF-β3(22/23)β1, pPLMu.TGF-β2(22/23)β3 and pPLMu.TGF-β3(22/23)β2, respectively, in order to express the coding sequences of mature TGF-β1(22/23)β2, TGF-β2(22/23)β1, TGF-β1(22/23)β3, TGF-β3(22/23)β1, TGF-β2(22/23)β3 and TGF-β3(22/23)β2.

## C. Transformation of S. cerevisiae strain GRF18

Saccharomyces cerevisiae strain GRF18 (MATα, his3-11, his3-15, leu2-3, leu2-112, can$^R$, DSM 3665) is transformed with plasmids pJDB207R/PH05-TGF-β1(44/45)β2, pJDB207R/PH05-TGF-β2(44/45)β1, pJDB207R/PH05-TGF-β1(44/45)β3, pJDB207R/PH05-TGF-β3(44/45)β1, pJDB207R/PH05-TGF-β2(44/45)β3 or pJDB207R/PH05-TGF-β3(44/45)β2, using the transformation protocol described by Hinnen, A. et al. (1978) PNAS 75, 1929. Transformed yeast cells are selected on yeast minimal medium plates deficient in leucine. Single transformed yeast colonies are isolated and referred to as

Saccharomyces cerevisiae GRF18/pJDB207R/PH05-TGF-β1(44/45)β2,
Saccharomyces cerevisiae GRF18/pJDB207R/PH05-TGF-β2(44/45)β1,
Saccharomyces cerevisiae GRF18/pJDB207R/PH05-TGF-β1(44/45)β3,
Saccharomyces cerevisiae GRF18/pJDB207R/PH05-TGF-β3(44/45)β1,
Saccharomyces cerevisiae GRF18/pJDB207R/PH05-TGF-β2(44/45)β3, and
Saccharomyces cerevisiae GRF18/pJDB207R/PH05-TGF-β3(44/45)β2.

In an analogous manner Saccharomyces cerevisiae strain GRF18 (MATα, his3-11, his3-15, leu2-3, leu2-112, can$^R$, DSM 3665) is transformed with plasmids pJDB207R/PH05-TGF-β1(56/57)β2, pJDB207R/PH05-TGF-β2(56/57)β1, pJDB207R/PH05-TGF-β1(56/57)β3, pJDB207R/PH05-TGF-β3(56/57)β1, pJDB207R/PH05-TGF-β2(56/57)β3 and pJDB207R/PH05-TGF-β3(56/57)β2. Transformed yeast cells are selected on yeast minimal medium plates deficient in leucine. Single transformed yeast colonies are isolated and referred to as

Saccharomyces cerevisiae GRF18/pJDB207R/PH05-TGF-β1(56/57)β2,
Saccharomyces cerevisiae GRF18/pJDB207R/PH05-TGF-β2(56/57)β1,
Saccharomyces cerevisiae GRF18/pJDB207R/PH05-TGF-β1(56/57)β3,

Saccharomyces cerevisiae GRF18/pJDB207R/PH05-TGF-β3(56/57)β1,
Saccharomyces cerevisiae GRF18/pJDB207R/PH05-TGF-β2(56/57)β3, and
Saccharomyces cerevisiae GRF18/pJDB207R/PH05-TGF-β3(56/57)β2.

In an analogous manner Saccharomyces cerevisiae strain GRF18 (MATα, his3-11, his3-15, leu2-3, leu2-112, can^R, DSM 3665) is transformed with plasmids pJDB207R/PH05-TGF-β1(79/80)β2, pJDB207R/PH05-TGF-β2(79/80)β1, pJDB207βH05-TGF-β1(79/80)β3, pJDB207R/PH05-TGF-β3(79/80)β1, pJDB207R/PH05-TGF-β2(79/80)β3 and pJDB207R/PH05-TGF-β3(79/80)β2. Transformed yeast cells are selected on yeast minimal medium plates deficient in leucine. Single transformed yeast colonies are isolated and referred to as
Saccharomyces cerevisiae GRF18/pJDB207R/PH05-TGF-β1(79/80)β2,
Saccharomyces cerevisiae GRF18/pJDB207R/PH05-TGF-β2(79/80)β1,
Saccharomyces cerevisiae GRF18/pJDB207R/PH05-TGF-β1(79/80)β3,
Saccharomyces cerevisiae GRF18/pJDB207R/PH05-TGF-β3(79/80)β1,
Saccharomyces cerevisiae GRF18/pJDB207R/PH05-TGF-β2(79/80)β3, and
Saccharomyces cerevisiae GRF18/pJDB207R/PH05-TGF-β3(79/80)β2.

In an analogous manner Saccharomyces cerevisiae strain GRF18 (MATα, his3-11, his3-15, leu2-3, leu2-112, can^R, DSM 3665) is transformed with plasmids pJDB207R/PH05-TGF-β1(90/91)β2, pJDB207R/PH05-TGF-β2(90/91)β1, pJDB207R/PH05-TGF-β1(90/91)β3, pJDB207R/PH05-TGF-β3(90/91)β1, pJDB207R/PH05-TGF-β2(90/91)β3 and pJDB207R/PH05-TGF-β3(90/91)β2. Transformed yeast cells are selected on yeast minimal medium plates deficient in leucine. Single transformed yeast colonies are isolated and referred to as
Saccharomyces cerevisiae GRF18/pJDB207R/PH05-TGF-β1(90/91)β2,
Saccharomyces cerevisiae GRF18/pJDB207R/PH05 -TGF-β2(90/91)β1,
Saccharomyces cerevisiae GRF18/pJDB207R/PH05-TGF-β1(90/91)β3,
Saccharomyces cerevisiae GRF18/pJDB207R/PH05-TGF-β3(90/91)β1,
Saccharomyces cerevisiae GRF18/pJDB207R/PH05-TGF-β2(90/91)β3 and
Saccharomyces cerevisiae GRF18/pJDB207R/PH05-TGF-β3(90/91)β2.

In an analogous manner Saccharomyces cerevisiae strain GRF18 (MATα, his3-11, his3-15, leu2-3, leu2-112, can^R, DSM 3665) is transformed with plasmids pJDB207R/PH05-TGF-β1(22/23)β2, pJDB207R/PH05-TGF-β2(22/23)β1, pJDB207R/PH05-TGF-β1(22/23)β3, pJDB 207R/PH05-TGF-β3(22/23)β1, pJDB207R/PH05-TGF-β2(22/23)β3 and pJDB207R/PH05-TGF-β3(22/23)β2. Transformed yeast cells are selected on yeast minimal medium plates deficient in leucine Single transformed yeast colonies are isolated and referred to as
Saccharomyces cerevisiae GRF18/pJDB207R/PH05-TGF-β1(22/23)β2,
Saccharomyces cerevisiae GRF18/pJDB207R/PH05-TGF-β2(22/23)β1,
Saccharomyces cerevisiae GRF18/pJDB207R/PH05-TGF-β1(22/23)β3,
Saccharomyces cerevisiae GRF18/pJDB207R/PH05-TGF-β3(22/23)β1,
Saccharomyces cerevisiae GRF18/pJDB207R/PH05-TGF-β2(22/23)β3 and
Saccharomyces cerevisiae GRF18/pJDB207R/PH05-TGF-β3(22/23)β2.

D. Fermentation of S. cerevisiae transformants and preparation of cell extracts

The yeast transformants, as mentioned above, contain plasmids with PH05 promoter-controlled expression cassettes and therefore require derepression of the promoter for the expression of the TGF-β hybrid molecules. Transformants are each grown in two successive precultures (10 ml and 50 ml) in yeast high $P_i$ minimal medium prepared according to the recipe of the Difco Yeast Nitrogen Base without amino acids but containing 10 g/l L-asparagine instead of $(NH_4)_2SO_4$, 1 g/l L-histidine and 20 g/l glucose. The cells of the second preculture are washed in 0.9 % NaCl and all the cells are used to inoculate 100 ml of low $P_i$ minimal medium prepared according to the recipe of the Difco Yeast Nitrogen Base medium (without amino acids), but containing 0.03 g/l $KH_2PO_4$, 10 g/l L-asparagine, 1 g/l L-histidine and 20 g/l glucose. The cultures are agitated at 30°C at 180 rpm.

Cells from 10 ml of culture are collected at 5 h, 24 h and 48 h by centrifugation at 3000 rpm and washed once in 0.9 % NaCl. The cell pellet is resuspended in lysis buffer [66 mM potassium phosphate pH 7.4, 4 mM Zwittergent (Calbiochem)]. 8 g of glass beads (0.5-0.75 mm in diameter) are added and the suspension is shaken vigorously 4-5 times for 2 min each on a Vortex Mixer in the cold. The cell extract is decanted to get rid of the glass beads. Cell debris in the extract are sedimented by centrifugation for 5 min at 3000 rpm at 4°C. The supernatant and pellets are separated and stored at -20°C.

Example 5: Production of dimeric, biologically active hybrid TGF-β proteins

The procedures given below∗ for the production of dimeric, biologically active TGF-β1(44/45)β2 hybrid may be applied in analogy for the recovery of other dimeric, biologically active TGF-β hybrid proteins.

E.coliLC137/pPLMu.TGF-β1(44/45)β/2 cells are fermented as described above and inclusion bodies are prepared as follows. Cell disruption and recovery of the inclusion bodies is performed at 4°C. About 18 g of wet cells are suspended in 60 ml of 0.1 M TRIS/HCl, 10 mM EDTA, 1 mM PMSF (Phenyl Methan Sulphonyl Fluoride), pH 8.3 (disruption buffer). The cells are passed two times through a Frenchpress (SLM Instruments, Inc.) according to the manufacturers instructions and the volume is brought to 200 ml with the disruption buffer. The suspension is centrifuged for 20 min at 15.000 g. The pellet obtained is suspended in 100 ml disruption buffer containing 1 M NaCl and centrifuged for 10 min as above. The pellet is suspended in 100 ml disruption buffer containing 1 % Triton X-100 (Pierce) and again centrifuged for 10 min as above.

0.3 g of the washed pellet is then suspended in 10 ml of 20 mM Tris/HCl, 1 mMEDTA, 1 mM PMSF, 0.1 % DTT, pH 8.0, and stirred with a magnetic stirrer for 1 h at room temperature. The sample is then brought to pH 2.5 with concentrated acetic acid and homogenised in a Teflon tissue homogenizer and centrifuged in a Centricon H-401 centrifuge (Kontron Instruments) with a fixed angle rotor A.8.24 for 60 min, at 15 °C and 12 000 rpm. The acetic acid of the clear supernatant is exchanged with 10 mM HCl in an Amicon 8010 stirred cell with YM05 filter by repeated concentration and dilution of the solution with 10 mM HCl.

Individual aliquots of the thus solubilized monomeric TGF-β1(44/45)β2 hybrid in 10 mM HCl are dried in vacuo and analyzed by SDS polyacrylamide gel electrophoresis under reducing conditions on 15 % polyacrylamide slab gels stained with Coomassie Blue R-250. A band of about 12 000 is obtained.

Another aliquot of the solublized TGF-β1(44/45)β2 hybrid is evaporated in vacuo, dissolved in 25 μl acetic acid and subjected to amino acid sequence determination in a gas phase sequencer model 470A (Applied Biosystems). The N-terminal amino acid sequence thus obtained corresponds with the N-terminus of TGF-β1 (see SEQ ID NO. 1).

6 ml of the solution of TGF-β1(44/45)β2 obtained above, i.e. 1.5 mg of the TGF-β1(44/45)β2, is mixed with 0.14 g CHAPS (Sigma C3023 Lot 80H5018) and precooled at 4 °C. The solution is then slowly added to 7.5 ml of Buffer I (precooled to 4 °C) consisting of 2 M NaCl, 0.2 M Tris, 30 mM CHAPS, 4 mM EDTA, 5 mM Glutathione reduced, pH 8.5. The mixture is stirred for 1 h at room temperature. Then 1.5 ml Buffer II consisitng of 0.1 M Tris, 10 mM Glutathione oxidized, pH 8.0, precooled at 4 °C, is added slowly. The mixture is kept at 4 °C to allow the hybrid TGF-β1(44/45)β2 to refold into the biologically active dimeric form.

After 260 h the refolding process is stopped by adding 4 N HCl to pH 2.5. The sample is diafiltered on an Amicon 8010 stirred cell with YM05 membrane against 10 mM HCl and concentrated to 5 ml and then centrifuged in a MSE table top centrifuge (model centaur 2) for 5 min at 3500 rpm. The clear solution is applied at a flow rate of 1 ml/min onto a Mono S HR 5/5 column equilibrated in buffer A (20 mM sodium acetate, 30 % isopropanol, pH 4.0) connected to a FPLC chromatography system (Pharmacia) with LCC 500 unit and a UV-MII Detector (Pharmacia) at a wavelength of 280 nm. A linear gradient over 30 min starting at injection time at equilibration conditions and ending with a mixture of 50 % buffer A (20 mM sodium acetate, 30 % Isopropanol, pH 4.0) and 50 % buffer B (corresponds to buffer A with 1 M NaCl).

Dimeric active TGF-β1(44/45)β2 is eluted about 8 min. after start of the gradient, collected and concentrated to 1 ml with a Millipore Ultrafree MC unit (5000 NMWL) in a Heraeus Christ Biofuge A at 10 000 rpm. TGF-β1(44/45)β2 is further purified by RP-HPLC using a system consisting of a Vydac C4 214TP5415 column (0.46 cm x 15 cm), two pumps (Waters 510) controlled by Data and Chromatography Control Station (Waters 840) and a UV Detector (Applied Biosystems, model 783) set at 216 nm. The column is equilibrated in 80 % solvent A (0.1 % TFA in water), 20 % solvent B (0.08 % TFA in acetonitrile). At injection time a linear gradient from 20 to 40 % solvent B in 40 min is applied. The peak with a retention time of 30.25 min is collected and analysed for biological activity as described hereinafter.

Example 6: In vitro activity tests for dimeric, biologically active hybrid TGF-β proteins

A. BALB/c-3T3 cell migration assay: Purified TGF-β hybrids are tested in this previously described assay (Bürk, R.R. (1973) Proc.Natl.Acad.Sci.USA 70:369-372) which measures the number of BALB/c 3T3 cells that have migrated from the monolayer in wounded cultures maintained in serum-free medium for 22 h in the presence or absence of a hybrid TGF-β.

B. Stimulation of AKR-2B cell DNA synthesis assay: Purified TGF-β hybrids are tested in this previously described assay (Graycar, J.L. et al., (1989) Molecular Endocrinology 3:1977-1986) which measures the increase in the levels of [3H]-Thymidine incorporation, over the last 16h of a 66h culture period, in cultures of AKR-2B cells maintained in McCoy's 5A medium containing 5% fetal bovine serum in the presence of a hybrid TGF-β.

C. Inhibition of CCL-64 cell DNA synthesis assay: Purified TGF-β hybrids are tested in a modified version of this previously described assay (Graycar, J.L. etal., (1989) Molecular Endocrinology 3:1977-1986) which measures the decrease in the levels of [3H]-Thymidine incorporation, over the last 16h of a 66h culture period, in cultures of CCL-64 cells maintained in DMEM medium containing 5% fetal bovine serum in the presence of a hybrid TGF-β.

Deposited Microorganisms:

Deposition of microorganisms

The following microorganisms were deposited at the Deutsche Sammlung von Mikroorganismen (DSM), Mascheroder Weg 1b, D-3300 Braunschweig (FRG):

| microorganism | deposition date | accession number |
|---|---|---|
| E. coli LC 137/pPLMu.hTGF-β1 | November 28,1989 | DSM 5656 |
| E. coli LC 137/pPLMu.hTGF-β2 | November 28,1989 | DSM 5657 |
| E. coli LC 137/pPLMu.hTGF-β3 | November 28,1989 | DSM 5658 |
| Saccharomyces cerevisiae GRF 18 | March 4, 1986 | DSM 3665 |
| E. coli HB101/p31R/SS-TPAΔ2 | October 23, 1987 | DSM 4295 |
| E. coli HB101/pJDB207 | November 7, 1991 | DSM 6782 |

Sequence listing

SEQ ID NO. 1

Sequence Type: Nucleotide with corresponding polypeptide

Sequence Length: 339 base pairs

Strandedness: double

Topology: linear

Source: human cDNA

Immediate experimental source: E. coli LC 137/pPLMu.hTGF-β1 (DSM 5656)

Features: from 1 to 336 coding region for TGF-β1

```
GCC CTG GAC ACC AAC TAT TGC TTC AGC TCC ACG GAG AAG        39
Ala Leu Asp Thr Asn Tyr Cys Phe Ser Ser Thr Glu Lys
                  5                   10


AAC TGC TGC GTG CGG CAG CTG TAC ATT GAC TTC CGC AAG        78
Asn Cys Cys Val Arg Gln Leu Tyr Ile Asp Phe Arg Lys
    15                  20                  25


GAC CTC GGC TGG AAG TGG ATC CAC GAG CCC AAG GGC TAC       117
Asp Leu Gly Trp Lys Trp Ile His Glu Pro Lys Gly Tyr
            30                  35


CAT GCC AAC TTC TGC CTC GGG CCC TGC CCC TAC ATT TGG       156
His Ala Asn Phe Cys Leu Gly Pro Cys Pro Tyr Ile Trp
 40                 45                  50


AGC CTG GAC ACG CAG TAC AGC AAG GTC CTG GCC CTG TAC       195
Ser Leu Asp Thr Gln Tyr Ser Lys Val Leu Ala Leu Tyr
        55                  60                  65


AAC CAG CAT AAC CCG GGC GCC TCG GCG GCG CCG TGC TGC       234
Asn Gln His Asn Pro Gly Ala Ser Ala Ala Pro Cys Cys
            70                  75
```

```
GTG CCG CAG GCG CTG GAG CCG CTG CCC ATC GTG TAC TAC        273
Val Pro Gln Ala Leu Glu Pro Leu Pro Ile Val Tyr Tyr
    80                      85                  90


GTG GGC CGC AAG CCC AAG GTG GAG CAG CTG TCC AAC ATG        312
Val Gly Arg Lys Pro Lys Val Glu Gln Leu Ser Asn Met
            95                  100


ATC GTG CGC TCC TGC AAG TGC AGC TGA                        339
Ile Val Arg Ser Cys Lys Cys Ser
105                 110
```

SEQ ID NO. 2

Sequence Type: Nucleotide with corresponding polypeptide

Sequence Length: 339 base pairs

Strandedness: double

Topology: linear

Source: human cDNA

Immediate experimental source: E. coli LC 137/pPLMu.hTGF-β2 (DSM 5657)

Features: from 1 to 336 coding region for TGF-β2

```
GCT TTG GAT GCG GCC TAT TGC TTT AGA AAT GTG CAG GAT        39
Ala Leu Asp Ala Ala Tyr Cys Phe Arg Asn Val Gln Asp
                5                   10


AAT TGC TGC CTA CGT CCA CTT TAC ATT GAT TTC AAG AGG        78
Asn Cys Cys Leu Arg Pro Leu Tyr Ile Asp Phe Lys Arg
    15                  20                  25


GAT CTA GGG TGG AAA TGG ATA CAC GAA CCC AAA GGG TAC        117
Asp Leu Gly Trp Lys Trp Ile His Glu Pro Lys Gly Tyr
            30                  35
```

```
AAT GCC AAC TTC TGT GCT GGA GCA TGC CCG TAT TTA TGG        156
Asn Ala Asn Phe Cys Ala Gly Ala Cys Pro Tyr Leu Trp
40                      45                  50


AGT TCA GAC ACT CAG CAC AGC AGG GTC CTG AGC TTA TAT        195
Ser Ser Asp Thr Gln His Ser Arg Val Leu Ser Leu Tyr
        55                  60                  65


AAT ACC ATA AAT CCA GAA GCA TCT GCT TCT CCT TGC TGC        234
Asn Thr Ile Asn Pro Glu Ala Ser Ala Ser Pro Cys Cys
                70                  75


GTG TCC CAA GAT TTA GAA CCT CTA ACC ATT CTC TAC TAC        273
Val Ser Gln Asp Leu Glu Pro Leu Thr Ile Leu Tyr Tyr
    80                  85                  90


ATT GGC AAA ACA CCC AAG ATT GAA CAG CTT TCT AAT ATG        312
Ile Gly Lys Thr Pro Lys Ile Glu Gln Leu Ser Asn Met
            95                  100


ATT GTA AAG TCT TGC AAA TGC AGC TAA                        339
Ile Val Lys Ser Cys Lys Cys Ser
105                 110
```

SEQ ID NO. 3

Sequence Type: Nucleotide with corresponding polypeptide

Sequence Length: 339 base pairs

Strandedness: double

Topology: linear

Source: human cDNA

Immediate experimental source: E. coli LC 137/pPLMu.hTGF-β3 (DSM 5658)

Features: from 1 to 336 coding region for TGF-β3

GCT TTG GAC ACC AAT TAC TGC TTC CGC AAC TTG GAG GAG     39
Ala Leu Asp Thr Asn Tyr Cys Phe Arg Asn Leu Glu Glu
                5                  10

AAC TGC TGT GTG CGC CCC CTC TAC ATT GAC TTC CGA CAG     78
Asn Cys Cys Val Arg Pro Leu Tyr Ile Asp Phe Arg Gln
        15             20             25

GAT CTG GGC TGG AAG TGG GTC CAT GAA CCT AAG GGC TAC    117
Asp Leu Gly Trp Lys Trp Val His Glu Pro Lys Gly Tyr
              30               35

TAT GCC AAC TTC TGC TCA GGC CCT TGC CCA TAC CTC CGC    156
Tyr Ala Asn Phe Cys Ser Gly Pro Cys Pro Tyr Leu Arg
40              45               50

AGT GCA GAC ACA ACC CAC AGC ACG GTG CTG GGA CTG TAC    195
Ser Ala Asp Thr Thr His Ser Thr Val Leu Gly Leu Tyr
        55             60             65

AAC ACT CTG AAC CCT GAA GCA TCT GCC TCG CCT TGC TGC    234
Asn Thr Leu Asn Pro Glu Ala Ser Ala Ser Pro Cys Cys
              70               75

GTG CCC CAG GAC CTG GAG CCC CTG ACC ATC CTG TAC TAT    273
Val Pro Gln Asp Leu Glu Pro Leu Thr Ile Leu Tyr Tyr
     80               85              90

GTT GGG AGG ACC CCC AAA GTG GAG CAG CTC TCC AAC ATG    312
Val Gly Arg Thr Pro Lys Val Glu Gln Leu Ser Asn Met
              95              100

GTG GTG AAG TCT TGT AAA TGT AGC TGA                339
Val Val Lys Ser Cys Lys Cys Ser
105               110

SEQ ID NO.4

Sequence Type: Nucleotide with corresponding polypeptide

Sequence length: 336 base pairs

Strandedness: double

Topology: linear

Source: human cDNA

Immediate experimental source: E.coli LC 137/pPLMu.TGF-ß1(44/45)β2

Features: from 1 to 336 coding region for TGF-ß1(44/45)β2 hybrid

```
GCC CTG GAC ACC AAC TAT TGC TTC AGC TCC ACG GAG AAG        39
Ala Leu Asp Thr Asn Tyr Cys Phe Ser Ser Thr Glu Lys
                  5                   10


AAC TGC TGC GTG CGG CAG CTG TAC ATT GAC TTC CGC AAG        78
Asn Cys Cys Val Arg Gln Leu Tyr Ile Asp Phe Arg Lys
        15                  20                  25


GAC CTC GGC TGG AAG TGG ATC CAC GAG CCC AAG GGC TAC       117
Asp Leu Gly Trp Lys Trp Ile His Glu Pro Lys Gly Tyr
                30                  35


CAT GCC AAC TTC TGT GCT GGA GCA TGC CCG TAT TTA TGG       156
His Ala Asn Phe Cys Ala Gly Ala Cys Pro Tyr Leu Trp
 40                  45                  50


AGT TCA GAC ACT CAG CAC AGC AGG GTC CTG AGC TTA TAT       195
Ser Ser Asp Thr Gln His Ser Arg Val Leu Ser Leu Tyr
          55                  60                  65


AAT ACC ATA AAT CCA GAA GCA TCT GCT TCT CCT TGC TGC       234
Asn Thr Ile Asn Pro Glu Ala Ser Ala Ser Pro Cys Cys
               70                  75
```

```
GTG TCC CAA GAT TTA GAA CCT CTA ACC ATT CTC TAC TAC        273
Val Ser Gln Asp Leu Glu Pro Leu Thr Ile Leu Tyr Tyr
        80                  85                  90


ATT GGC AAA ACA CCC AAG ATT GAA CAG CTT TCT AAT ATG        312
Ile Gly Lys Thr Pro Lys Ile Glu Gln Leu Ser Asn Met
            95                  100


ATT GTA AAG TCT TGC AAA TGC AGC                            336
Ile Val Lys Ser Cys Lys Cys Ser
105                  110
```

SEQ ID NO.5

Sequence Type: Nucleotide with corresponding polypeptide

Sequence length: 336 base pairs

Strandedness: double

Topology: linear

Source: human cDNA

Immediate experimental source: E.coli LC 137/pPLMu.TGF-ß1(44/45)β3

Features: from 1 to 336 coding region for TGF-ß1(44/45)β3 hybrid

```
GCC CTG GAC ACC AAC TAT TGC TTC AGC TCC ACG GAG AAG         39
Ala Leu Asp Thr Asn Tyr Cys Phe Ser Ser Thr Glu Lys
                5                  10


AAC TGC TGC GTG CGG CAG CTG TAC ATT GAC TTC CGC AAG         78
Asn Cys Cys Val Arg Gln Leu Tyr Ile Asp Phe Arg Lys
        15                  20                  25


GAC CTC GGC TGG AAG TGG ATC CAC GAG CCC AAG GGC TAC        117
Asp Leu Gly Trp Lys Trp Ile His Glu Pro Lys Gly Tyr
            30                  35
```

```
CAT GCC AAC TTC TGC TCA GGC CCT TGC CCA TAC CTC CGC        156
His Ala Asn Phe Cys Ser Gly Pro Cys Pro Tyr Leu Arg
 40                  45                  50


AGT GCA GAC ACA ACC CAC AGC ACG GTG CTG GGA CTG TAC        195
Ser Ala Asp Thr Thr His Ser Thr Val Leu Gly Leu Tyr
         55                  60                  65


AAC ACT CTG AAC CCT GAA GCA TCT GCC TCG CCT TGC TGC        234
Asn Thr Leu Asn Pro Glu Ala Ser Ala Ser Pro Cys Cys
                 70                  75


GTG CCC CAG GAC CTG GAG CCC CTG ACC ATC CTG TAC TAT        273
Val Pro Gln Asp Leu Glu Pro Leu Thr Ile Leu Tyr Tyr
         80                  85                  90


GTT GGG AGG ACC CCC AAA GTG GAG CAG CTC TCC AAC ATG        312
Val Gly Arg Thr Pro Lys Val Glu Gln Leu Ser Asn Met
                 95                  100


GTG GTG AAG TCT TGT AAA TGT AGC                            336
Val Val Lys Ser Cys Lys Cys Ser
105                  110
```

SEQ ID NO.6

Sequence Type: Nucleotide with corresponding polypeptide

Sequence length: 336 base pairs

Strandedness: double

Topology: linear

Source: human cDNA

Immediate experimental source: E.coli LC 137/pPLMu.TGF-ß2(44/45)ß1

Features: from 1 to 336 coding region for TGF-ß2(44/45)ß1 hybrid

```
GCT TTG GAT GCG GCC TAT TGC TTT AGA AAT GTG CAG GAT        39
Ala Leu Asp Ala Ala Tyr Cys Phe Arg Asn Val Gln Asp
                     5                   10


AAT TGC TGC CTA CGT CCA CTT TAC ATT GAT TTC AAG AGG        78
Asn Cys Cys Leu Arg Pro Leu Tyr Ile Asp Phe Lys Arg
     15                  20                  25


GAT CTA GGG TGG AAA TGG ATA CAC GAA CCC AAA GGG TAC        117
Asp Leu Gly Trp Lys Trp Ile His Glu Pro Lys Gly Tyr
             30                  35


AAT GCC AAC TTC TGC CTC GGG CCC TGC CCC TAC ATT TGG        156
Asn Ala Asn Phe Cys Leu Gly Pro Cys Pro Tyr Ile Trp
 40                  45                  50


AGC CTG GAC ACG CAG TAC AGC AAG GTC CTG GCC CTG TAC        195
Ser Leu Asp Thr Gln Tyr Ser Lys Val Leu Ala Leu Tyr
             55                  60                  65


AAC CAG CAT AAC CCG GGC GCC TCG GCG GCG CCG TGC TGC        234
Asn Gln His Asn Pro Gly Ala Ser Ala Ala Pro Cys Cys
                 70                  75


GTG CCG CAG GCG CTG GAG CCG CTG CCC ATC GTG TAC TAC        273
Val Pro Gln Ala Leu Glu Pro Leu Pro Ile Val Tyr Tyr
     80                  85                  90


GTG GGC CGC AAG CCC AAG GTG GAG CAG CTG TCC AAC ATG        312
Val Gly Arg Lys Pro Lys Val Glu Gln Leu Ser Asn Met
             95                  100


ATC GTG CGC TCC TGC AAG TGC AGC                            336
Ile Val Arg Ser Cys Lys Cys Ser
105                  110
```

SEQ ID NO.7

Sequence Type: Nucleotide with corresponding polypeptide

Sequence length: 336 base pairs

Strandedness: double

Topology: linear

Source: human cDNA

Immediate experimental source: E.coli LC 137/pPLMu.TGF-ß2(44/45)β3

Features: from 1 to 336 coding region for TGF-ß2(44/45)β3 hybrid

```
GCT TTG GAT GCG GCC TAT TGC TTT AGA AAT GTG CAG GAT      39
Ala Leu Asp Ala Ala Tyr Cys Phe Arg Asn Val Gln Asp
                      5                   10

AAT TGC TGC CTA CGT CCA CTT TAC ATT GAT TTC AAG AGG      78
Asn Cys Cys Leu Arg Pro Leu Tyr Ile Asp Phe Lys Arg
     15              20                  25

GAT CTA GGG TGG AAA TGG ATA CAC GAA CCC AAA GGG TAC     117
Asp Leu Gly Trp Lys Trp Ile His Glu Pro Lys Gly Tyr
             30              35

AAT GCC AAC TTC TGC TCA GGC CCT TGC CCA TAC CTC CGC     156
Asn Ala Asn Phe Cys Ser Gly Pro Cys Pro Tyr Leu Arg
 40              45                  50

AGT GCA GAC ACA ACC CAC AGC ACG GTG CTG GGA CTG TAC     195
Ser Ala Asp Thr Thr His Ser Thr Val Leu Gly Leu Tyr
         55              60                  65

AAC ACT CTG AAC CCT GAA GCA TCT GCC TCG CCT TGC TGC     234
Asn Thr Leu Asn Pro Glu Ala Ser Ala Ser Pro Cys Cys
             70                  75
```

```
GTG CCC CAG GAC CTG GAG CCC CTG ACC ATC CTG TAC TAT     273
Val Pro Gln Asp Leu Glu Pro Leu Thr Ile Leu Tyr Tyr
    80                      85                  90

GTT GGG AGG ACC CCC AAA GTG GAG CAG CTC TCC AAC ATG     312
Val Gly Arg Thr Pro Lys Val Glu Gln Leu Ser Asn Met
            95                  100

GTG GTG AAG TCT TGT AAA TGT AGC                         336
Val Val Lys Ser Cys Lys Cys Ser
105                 110
```

SEQ ID NO.8

Sequence Type: Nucleotide with corresponding polypeptide

Sequence length: 336 base pairs

Strandedness: double

Topology: linear

Source: human cDNA

Immediate experimental source: E.coli LC 137/pPLMu.TGF-ß3(44/45)β1

Features: from 1 to 336 coding region for TGF-ß3(44/45)β1 hybrid

```
GCT TTG GAC ACC AAT TAC TGC TTC CGC AAC TTG GAG GAG     39
Ala Leu Asp Thr Asn Tyr Cys Phe Arg Asn Leu Glu Glu
                5                       10

AAC TGC TGT GTG CGC CCC CTC TAC ATT GAC TTC CGA CAG     78
Asn Cys Cys Val Arg Pro Leu Tyr Ile Asp Phe Arg Gln
    15                  20                      25

GAT CTG GGC TGG AAG TGG GTC CAT GAA CCT AAG GGC TAC     117
Asp Leu Gly Trp Lys Trp Val His Glu Pro Lys Gly Tyr
            30                  35
```

```
TAT GCC AAC TTC TGC CTC GGG CCC TGC CCC TAC ATT TGG        156
Tyr Ala Asn Phe Cys Leu Gly Pro Cys Pro Tyr Ile Trp
 40                      45                  50


AGC CTG GAC ACG CAG TAC AGC AAG GTC CTG GCC CTG TAC        195
Ser Leu Asp Thr Gln Tyr Ser Lys Val Leu Ala Leu Tyr
         55                  60                  65


AAC CAG CAT AAC CCG GGC GCC TCG GCG GCG CCG TGC TGC        234
Asn Gln His Asn Pro Gly Ala Ser Ala Ala Pro Cys Cys
                 70                  75


GTG CCG CAG GCG CTG GAG CCG CTG CCC ATC GTG TAC TAC        273
Val Pro Gln Ala Leu Glu Pro Leu Pro Ile Val Tyr Tyr
     80                  85                  90


GTG GGC CGC AAG CCC AAG GTG GAG CAG CTG TCC AAC ATG        312
Val Gly Arg Lys Pro Lys Val Glu Gln Leu Ser Asn Met
             95                  100


ATC GTG CGC TCC TGC AAG TGC AGC                            336
Ile Val Arg Ser Cys Lys Cys Ser
105                 110
```

SEQ ID NO.9

Sequence Type: Nucleotide with corresponding polypeptide

Sequence length: 336 base pairs

Strandedness: double

Topology: linear

Source: human cDNA

Immediate experimental source: E.coli LC 137/pPLMu.TGF-ß3(44/45)ß2

Features: from 1 to 336 coding region for TGF-ß3(44/45)ß2 hybrid

```
GCT TTG GAC ACC AAT TAC TGC TTC CGC AAC TTG GAG GAG          39
Ala Leu Asp Thr Asn Tyr Cys Phe Arg Asn Leu Glu Glu
                  5                    10

AAC TGC TGT GTG CGC CCC CTC TAC ATT GAC TTC CGA CAG          78
Asn Cys Cys Val Arg Pro Leu Tyr Ile Asp Phe Arg Gln
         15                  20                  25

GAT CTG GGC TGG AAG TGG GTC CAT GAA CCT AAG GGC TAC         117
Asp Leu Gly Trp Lys Trp Val His Glu Pro Lys Gly Tyr
             30                  35

TAT GCC AAC TTC TGT GCT GGA GCA TGC CCG TAT TTA TGG         156
Tyr Ala Asn Phe Cys Ala Gly Ala Cys Pro Tyr Leu Trp
 40                  45                  50

AGT TCA GAC ACT CAG CAC AGC AGG GTC CTG AGC TTA TAT         195
Ser Ser Asp Thr Gln His Ser Arg Val Leu Ser Leu Tyr
         55                  60                  65

AAT ACC ATA AAT CCA GAA GCA TCT GCT TCT CCT TGC TGC         234
Asn Thr Ile Asn Pro Glu Ala Ser Ala Ser Pro Cys Cys
                  70                  75

GTG TCC CAA GAT TTA GAA CCT CTA ACC ATT CTC TAC TAC         273
Val Ser Gln Asp Leu Glu Pro Leu Thr Ile Leu Tyr Tyr
         80                  85                  90

ATT GGC AAA ACA CCC AAG ATT GAA CAG CTT TCT AAT ATG         312
Ile Gly Lys Thr Pro Lys Ile Glu Gln Leu Ser Asn Met
             95                  100

ATT GTA AAG TCT TGC AAA TGC AGC                             336
Ile Val Lys Ser Cys Lys Cys Ser
105                  110
```

SEQ ID NO. 10

Sequence Type: Nucleotide

Sequence length: 29 bases

Strandedness: single

Topology: linear

Source: synthetic oligonucleotide

Features: Oligo No. 1; TGF-β1 sense strand, includes NcoI site and represents the 5' end of the sequence encoding the mature peptide.

```
TCCCGGCACA  CCATGGCCCT  GGACACCAA        29
```

SEQ ID NO. 11

Sequence Type: Nucleotide

Sequence length: 27 bases

Strandedness: single

Topology: linear

Source: synthetic oligonucleotide

Features: Oligo No. 2; TGF-β1 antisense strand, includes SalI site and represents the 3' end of the sequence encoding the mature peptide.

```
CGGGGCGTCG  ACTCAGCTGC  ACTTGCA          27
```

SEQ ID NO. 12

Sequence Type: Nucleotide

Sequence length: 30 bases

Strandedness: single

Topology: linear

Source: synthetic oligonucleotide

Features: Oligo No. 3; TGF-β2 sense strand, includes NcoI site and represents the 5' end of the sequence encoding the mature peptide.

CGGCGGAAGA CCATGGCTTT GGATGCGGCC            30

SEQ ID NO. 13

Sequence Type: Nucleotide

Sequence length: 27 bases

Strandedness: single

Topology: linear

Source: synthetic oligonucleotide

Features: Oligo No. 4; TGF-β2 antisense strand, includes SalI site and represents the 3' end of the sequence encoding the mature peptide.

TTTCCAGTCG ACTTAGCTGC ATTTGCA            27

SEQ ID NO. 14

Sequence Type: Nucleotide

Sequence length: 29 bases

Strandedness: single

Topology: linear

Source: synthetic oligonucleotide

Features: Oligo No. 5; TGF-β3 sense strand, includes NcoI site and represents the 5' end of the sequence encoding the mature peptide.

CAGAGGAAGA CCATGGCTTT GGACACCAA            29

SEQ ID NO. 15
Sequence Type: Nucleotide
Sequence length: 27 bases
Strandedness: single
Topology: linear
Source: synthetic oligonucleotide
Features: Oligo No. 6; TGF-β3 antisense strand, includes SalI site and represents the 3' end of the sequence encoding the mature peptide.

GCACGTGTCG ACTCAGCTAC ATTTACA 27

SEQ ID NO.16
Sequence Type: Nucleotide
Sequence length: 21 bases
Strandedness: single
Topology: linear
Source: synthetic oligonucleotide
Features: Oligo No. 7; TGF-β1 sense strand, nucleotides 111 - 131.

GGGCTACCAT GCCAACTTCT G 21

SEQ ID NO. 17
Sequence Type: Nucleotide
Sequence length: 21 bases
Strandedness: single
Topology: linear
Source: synthetic oligonucleotide
Features: Oligo No. 8; TGF-β1 antisense strand, nucleotides 111 - 131.

CAGAAGTTGG CATGGTAGCC C 21

SEQ ID NO. 18

Sequence Type: Nucleotide

Sequence length: 21 bases

Strandedness: single

Topology: linear

Source: synthetic oligonucleotide

Features: Oligo No. 9; TGF-β3 sense strand, nucleotides 111 - 131.


GGGCTACTAT GCCAACTTCT G                    21


SEQ ID NO. 19

Sequence Type: Nucleotide

Sequence length: 21 bases

Strandedness: single

Topology: linear

Source: synthetic oligonucleotide

Features: Oligo No. 10; TGF-β3 antisense strand, nucleotides 111 - 131.


CAGAAGTTGG CATAGTAGCC C                    21


SEQ ID NO. 20

Sequence Type: Nucleotide

Sequence length: 21 bases

Strandedness: single

Topology: linear

Source: synthetic oligonucleotide

Features: Oligo No. 11; TGF-β2 sense strand, nucleotides 111 - 131.


AGGGTACAAT GCCAACTTCT G                    21

SEQ ID NO. 21
Sequence Type: Nucleotide
Sequence length: 21 base
Strandedness: single
Topology: linear
Source: synthetic oligonucleotide
Features: Oligo No. 12; TGF-β2 antisense strand, nucleotides 111 - 131.


CAGAAGTTGG CATTGTACCC T                    21


SEQ ID NO. 22
Sequence Type: Nucleotide
Sequence length: 21 bases
Strandedness: single
Topology: linear
Source: synthetic oligonucleotide
Features: Oligo No. 13; TGF-β1 sense strand, nucleotides 48 - 68.


CGTGCGGCAG CTGTACATTG A                    21


SEQ ID NO. 23
Sequence Type: Nucleotide
Sequence length: 21 bases
Strandedness: single
Topology: linear
Source: synthetic oligonucleotide
Features: Oligo No. 14; TGF-β1 antisense strand, nucleotides 68 - 48.


TCAATGTACA GCTGCCGCAC G                    21

SEQ ID NO. 24

Sequence Type: Nucleotide

Sequence length: 21 bases

Strandedness: single

Topology: linear

Source: synthetic oligonucleotide

Features: Oligo No. 15; TGF-β2 sense strand, nucleotides 48 - 68.


CCTACGTCCA CTTTACATTG A


SEQ ID NO. 25

Sequence Type: Nucleotide

Sequence length: 21 bases

Strandedness: single

Topology: linear

Source: synthetic oligonucleotide

Features: Oligo No. 16; TGF-β2 antisense strand, nucleotides 68 - 48.


TCAATGTAAA GTGGACGTAG G                    21


SEQ ID NO. 26

Sequence Type: Nucleotide

Sequence length: 21 bases

Strandedness: single

Topology: linear

Source: synthetic oligonucleotide

Features: Oligo No. 17; TGF-β3 sense strand, nucleotides 48 - 68.


TGTGCGCCCC CTCTACATTG A                    21

SEQ ID NO. 27

Sequence Type: Nucleotide

Sequence length: 21 bases

Strandedness: single

Topology: linear

Source: synthetic oligonucleotide

Features: Oligo No. 18; TGF-β3 antisense strand, nucleotides 68 - 48.


TCAATGTAGA GGGGGCGCAC A                   21


SEQ ID NO. 28

Sequence Type: Nucleotide

Sequence length: 26 bases

Strandedness: single

Topology: linear

Source: synthetic oligonucleotide

Features: Oligo No. 19; TGF-β1 sense strand, nucleotides 142-167.


TGCCCCTACA TTTGGAGCCT GGACAC                   26


SEQ ID NO. 29

Sequence Type: Nucleotide

Sequence length: 26 bases

Strandedness: single

Topology: linear

Source: synthetic oligonucleotide

Features: Oligo No. 20; TGF-β1 antisense strand, nucleotides 167-142.


GTGTCCAGGC TCCAAATGTA GGGGCA                   26

SEQ ID NO. 30

Sequence Type: Nucleotide

Sequence length: 26 bases

Strandedness: single

Topology: linear

Source: synthetic oligonucleotide

Features: Oligo No. 21; TGF-β2 sense strand, nucleotides 142-167.


TGCCCGTATT TATGGAGTTC AGACAC          26


SEQ ID NO. 31

Sequence Type: Nucleotide

Sequence length: 26 bases

Strandedness: single

Topology: linear

Source: synthetic oligonucleotide

Features: Oligo No. 22; TGF-β2 antisense strand, nucleotides 167-142.


GTGTCTGAAC TCCATAAATA CGGGCA          26


SEQ ID NO. 32

Sequence Type: Nucleotide

Sequence length: 26 bases

Strandedness: single

Topology: linear

Source: synthetic oligonucleotide

Features: Oligo No. 23; TGF-β3 sense strand, nucleotides 142-167.


TGCCCATACC TCTGTAGTGC AGACAC          26

SEQ ID NO. 33

Sequence Type: Nucleotide

Sequence length: 26 bases

Strandedness: single

Topology: linear

Source: synthetic oligonucleotide

Features: Oligo No. 24; TGF-β3 antisense strand, nucleotides 167-142.


GTGTCTGAAC TGCGGAGGTA TGGTCA                    26


SEQ ID NO. 34

Sequence Type: Nucleotide

Sequence length: 21 bases

Strandedness: single

Topology: linear

Source: synthetic oligonucleotide

Features: Oligo No. 25; TGF-β1 sense strand, nucleotides 217-237.


TCGGCGGCGC CGTGCTGCGT G                    21


SEQ ID NO. 35

Sequence Type: Nucleotide

Sequence length: 21 bases

Strandedness: single

Topology: linear

Source: synthetic oligonucleotide

Features: Oligo No. 26; TGF-β1 antisense strand, nucleotides 237-217.


CACGCAGCAC GGCGCCGCCG A                    21

SEQ ID NO. 36

Sequence Type: Nucleotide

Sequence length: 21 bases

Strandedness: single

Topology: linear

Source: synthetic oligonucleotide

Features: Oligo No. 27; TGF-β2 sense strand, nucleotides 217-237.


TCTGCTTCTC CTTGCTGCGT G                    21


SEQ ID NO. 37

Sequence Type: Nucleotide

Sequence length: 21 bases

Strandedness: single

Topology: linear

Source: synthetic oligonucleotide

Features: Oligo No. 28; TGF-β2 antisense strand, nucleotides 237-217.


CACGCAGCAA GGAGAAGCAG A                    21


SEQ ID NO. 38

Sequence Type: Nucleotide

Sequence length: 21 bases

Strandedness: single

Topology: linear

Source: synthetic oligonucleotide

Features: Oligo No. 29; TGF-β3 sense strand, nucleotides 217-237.


TCTGCCTCGC CTTGCTGCGT G                    21

SEQ ID NO. 39

Sequence Type: Nucleotide

Sequence length: 21 bases

Strandedness: single

Topology: linear

Source: synthetic oligonucleotide

Features: Oligo No. 30; TGF-β3 antisense strand, nucleotides 237-217.


CACGCAGCAA  GGCGAGGCAG  A                    21


SEQ ID NO. 40

Sequence Type: Nucleotide

Sequence length: 21 bases

Strandedness: single

Topology: linear

Source: synthetic oligonucleotide

Features: Oligo No. 31; TGF-β1 sense strand, nucleotides 252-272.


GCCGCTGCCC  ATCGTGTACT  A                    21


SEQ ID NO. 41

Sequence Type: Nucleotide

Sequence length: 21 bases

Strandedness: single

Topology: linear

Source: synthetic oligonucleotide

Features: Oligo No. 32; TGF-β1 antisense strand, nucleotides 272-252.


TAGTACACGA  TGGGCAGCGG  C                    21

SEQ ID NO. 42

Sequence Type: Nucleotide

Sequence length: 21 bases

Strandedness: single

Topology: linear

Source: synthetic oligonucleotide

Features: Oligo No. 33; TGF-β2 sense strand, nucleotides 252-272.


ACCTCTAACC ATTCTCTACT A                    21


SEQ ID NO. 43

Sequence Type: Nucleotide

Sequence length: 21 bases

Strandedness: single

Topology: linear

Source: synthetic oligonucleotide

Features: Oligo No. 34; TGF-β2 antisense strand, nucleotides 272-252.


TAGTAGAGAA TGGTTAGAGG T                    21


SEQ ID NO. 44

Sequence Type: Nucleotide

Sequence length: 21 bases

Strandedness: single

Topology: linear

Source: synthetic oligonucleotide

Features: Oligo No. 35; TGF-β3 sense strand, nucleotides 252-272.


GCCCTGACCA TCCTGTACT A                    21

SEQ ID NO. 45

Sequence Type: Nucleotide

Sequence length: 21 bases

Strandedness: single

Topology: linear

Source: synthetic oligonucleotide

Features: Oligo No. 36; TGF-β3 antisense strand, nucleotides 272-252.


TAGTACAGGA TGGTCAGGGG C                21


SEQ ID NO. 46

Sequence Type: Nucleotide

Sequence length: 34 bases

Strandedness: single

Topology: linear

Source: synthetic oligonucleotide

Features: Oligonucleotide I-1, encodes part of the invertase signal sequence


AATTCATGCT TTTGCAAGCT TTCCTTTTCC TTTT                34


SEQ ID NO. 47

Sequence Type: Nucleotide

Sequence length: 35 bases

Strandedness: single

Topology: linear

Source: synthetic oligonucleotide

Features: Oligonucleotide 1-2, complemetary to Oligonucleotide I-1


CAGCCAAAAG GAAAAGGAAA GCTTGCAAAA GCATG

35

SEQ ID NO. 48

Sequence Type: Nucleotide

Sequence length: 38 bases

Strandedness: single

Topology: linear

Source: synthetic oligonucleotide

Features: Oligonucleotide I-3, encodes part of the invertase signal sequence

```
GGCTGGTTTT GCAGCCAAAA TATCTGCATC TTAGCGTC
38
```

SEQ ID NO. 49

Sequence Type: Nucleotide

Sequence length: 37 bases

Strandedness: single

Topology: linear

Source: synthetic oligonucleotide

Features: Oligonucleotide I-4, complemetary to Oligonucleotide I-3

```
TCGAGACGCT AAGATGCAGA TATTTTGGCT GCAAAAC
37
```

FD4.4/BO

## Claims

1. A hybrid TGF-β molecule consisting of two or more parts, said parts consisting of contiguous stretches of 6 or more amino acids of a sequence which occurs in mature TGF-β isoforms.

2. A hybrid TGF-β molecule according to claim 1 consisting of 2, 3, 4, 5 or 6 parts.

3. A hybrid TGF-β molecule according to claim 1 consisting of two parts.

4. A hybrid TGF-β molecule according to claim 3 consisting of parts of the TGF-β isoforms human TGF-β1, human TGF-β2 and human TGF-β3 with the amino acid sequences depicted the Sequence Listing under SEQ ID NO. 1 to 3.

5. A hybrid TGF-β molecule according to claim 1 having a hinge point between parts derived from different parent TGF-β isoforms selected from the group of hinge points between amino acids 44 and 45, 56 and 57, 79 and 80, 90 and 91, and 22 and 23.

6. A hybrid TGF-β molecule according to claim 1 selected from the group consisting of the hybrids consisting of a N-terminal part of TGF-β1 and a C-terminal part of TGF-β2, a N-terminal part of TGF-β2 and a C-terminal part of TGF-β1, a N-terminal part of TGF-β1 and a C-terminal part of TGF-β3, a N-terminal part of TGF-β3 and a C-terminal part of TGF-β1, a N-terminal part of TGF-β2 and a C-terminal part of TGF-β3, and a N-terminal part of TGF-β3 and a C-terminal part of TGF-β2.

7. A hybrid TGF-β molecule according to claim 1 selected from the group consisting of the hybrids TGF-β1(44/45)β2, TGF-β2(44/45)β1, TGF-β1(44/45)β3, TGF-β3(44/45)β1, TGF-β2(44/45)β3, TGF-β3(44/45)β2, TGF-β1(56/57)β2, TGF-β2(56/57)β1, TGF-β1(56/57)β3, TGF-β3(56/57)β1, TGF-β2(56/57)β3, TGF-β3(56/57)β2, TGF-β1(79/80)β2, TGF-β2(79/80)β1, TGF-β1(79/80)β3, TGF-β3(79/80)β1, TGF-β3(79/80)β2, TGF-β2(79/80)β3, TGF-β1(90/91)β2, TGF-β2(90/91)β1, TGF-β1(90/91)β3, TGF-β3(90/91)β1, TGF-β3(90/91)β2, TGF-β2(90/91)β3, TGF-β1(22/23)β2, TGF-β2(22/23)β1, TGF-β1(22/23)β3, TGF-β3(22/23)β1, TGF-β3(22/23)β2, and TGF-β2(22/23)β3.

8. A hybrid TGF-β molecule according to claim 1 selected from the group consisting of the hybrids TGF-β1(44/45)β2, TGF-β2(44/45)β1, TGF-β1(44/45)β3, TGF-β3(44/45)β1, and TGF-β2(44/45)β3, TGF-β3(44/45)β2 depicted in the sequence listing under SEQ ID NOs. 4 to 9.

9. The hybrid TGF-β molecule according to claim 1 which is TGF-β3(44/45)β2 depicted in the sequence listing under SEQ ID NO. 9.

10. A recombinant DNA molecule encoding a hybrid TGF-β molecule according to claim 1.

11. A recombinant DNA molecule according to claim 10 which is an expression vector for the preparation of a hybrid TGF-β molecule.

12. A method for the preparation of a DNA molecule according to claim 10.

13. A host transformed with a DNA molecule according to claim 10.

14. A method for the preparation of a hybrid TGF-β molecule according to claim 1.

15. A pharmaceutical composition comprising a hybrid TGF-β molecule according to claim 1.

## EUROPEAN SEARCH REPORT

**European Patent Office**

Application Number

EP 92 81 0845

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 374 044 (ONCOGENE LIMITED PARTNERSHIP) <br> * page 5, line 5 - line 33 * | 1-15 | C12N15/12 <br> C12N15/62 <br> A61K37/02 <br> C12N1/21 <br> //(C12N1/21, <br> C12R1:865) |
| D,A | EP-A-0 433 225 (CIBA-GEIGY A.G.) <br> * the whole document * | 1-15 | |
| A | EP-A-0 376 785 (ONCOGENE LIMITED PARTNERSHIP) <br> * page 19, line 45 - page 21, line 21; figure 1C * | 1-15 | |
| D,A | EP-A-0 267 463 (ONCOGENE SCIENCE, INC.) <br> * page 41, line 54 - page 42, line 5; claims; figure 31 * | 1-15 | |
| P,X | PROC. NATL. ACAD. SCI. U. S. A. <br> vol. 89, no. 14, 15 July 1992, <br> pages 6290 - 6294 <br> QIAN, S.W. ET AL. 'Identification of a structural domain that distinguishes the actions of the type 1 and 2 isoforms of transforming growth factor beta on endothelial cells' <br> * the whole document * | 1-2,4, 10-14 | |

TECHNICAL FIELDS SEARCHED (Int. Cl.5)

C07K
C12N
A61K

The present search report has been drawn up for all claims

| Place of search | Date of completion of the search | Examiner |
|---|---|---|
| THE HAGUE | 01 FEBRUARY 1993 | ANDRES S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)